# EUROPEAN PATENT APPLICATION

(11) **EP 4 685 479 A1**
(43) Date of publication of application: **28.01.2026**
(21) Application number: 24774984.9
(22) Date of filing: 22.03.2024
(51) Int. Cl.: G01N 30/88, C12Q 1/28, G01N 30/02, G01N 30/06, G01N 30/26, G01N 30/34, G01N 30/74, G01N 33/66, G01N 33/72

(54) **BLOOD ANALYSIS DEVICE AND BLOOD ANALYSIS METHOD**

(30) Priority: 23.03.2023 JP 2023046792; 05.09.2023 JP 2023143702
(71) Applicant: Sekisui Medical Co., Ltd., Tokyo 103-0027 (JP)
(72) Inventor: SHIINA Shouta, Tokyo 103-0027 (JP); TAIRA Hiroaki, Tokyo 103-0027 (JP)
(74) Representative: Ter Meer Steinmeister & Partner
(86) International application number: PCT/JP2024/011205
(87) International publication number: WO 2024/195850

(57) **Abstract**

This blood analysis device (1) includes an introduction section (11) into which a blood sample is introduced, a first reagent holder (12) in which a first reagent for measuring a first measurement item of the blood sample is stored, a first pump (18) that controls liquid delivery of the first reagent, a second reagent holder (13) in which a second reagent for measuring a second measurement item of the blood sample is stored, a liquid delivery control mechanism (14) which is connected to the introduction section (11), the first pump (18) and the second reagent holder (13), and controls likquid delivery of the blood sample and the second reagent, a first line (15) which is connected to the liquid delivery control mechanism (14) and into which at least a portion of the blood sample is introduced, a liquid chromatography column (16) which is connected to the first line (15), and a flow cell optical detector (17) which is connected to the liquid chromatography column (16) and measures the first measurement item and the second measurement item.

## Description

### Technical Field

The present invention relates to a blood analysis device and a blood analysis method.

Priority is claimed on Japanese Patent Application No. 2023-046792, filed March 23, 2023, and Japanese Patent Application No. 2023-143702, filed September 5, 2023, the contents of which are incorporated herein by reference.

### Background Art

Hemoglobin binds nonenzymatically to glucose and metabolites thereof that are present in blood to form glycohemoglobin. Hemoglobin A1 is a glycohemoglobin of hemoglobin A which accounts for the majority of total hemoglobin. Hemoglobin A1 can be further classified into hemoglobin A1a, A1b and A1c. Hemoglobin A1c (hereinafter sometimes abbreviated as HbA1c) can continue to exist in the blood for the approximately 120 days that represent the lifespan of red blood cells. Because the glycation reaction rate for hemoglobin is slow, the ratio of glycohemoglobin to total hemoglobin (hereinafter sometimes referred to as simply the glycohemoglobin % or HbA1c%) is not strongly affected by temporary increases in the blood glucose level caused by food consumption or the like. Accordingly, by measuring HbA1c%, the blood glucose state across the past one to two months can be estimated. For this reason, HbA1c% can be used to ascertain changes in the blood glucose level over comparatively long periods, and is therefore clinically significant.

In their diabetes treatment guidelines from 2019, The Japan Diabetes Society describes methods for confirming diabetes that can be used as methods for diagnosing diabetes. Diabetes can be confirmed by either (1) blood glucose level, or (2) HbA1c. Specifically, a diagnosis of diabetes can be established when (1) the blood glucose level, reported as a fasting blood glucose level, is 126 mg/dL or higher, or the blood glucose level two hours after a glucose tolerance test is 200 mg/dL or higher, or the random blood glucose level is 200 mg/dL or higher, or when (2) HbA1c, reported as HbA1c%, is 6.5% or higher. Confirmation of the diabetes diagnosis is conducted twice, and at least one of these diagnoses must be confirmed by blood glucose level.

Patent Documents 1 and 2, disclose blood analysis devices capable of measuring both HbA1c% and the blood glucose level.

### Citation List

### Patent Documents

Patent Document 1: International Patent Publication 2012/043444
Patent Document 2: Japanese Unexamined Patent Application, First Publication No. 2014-95715

### Summary of Invention

### Technical Problem

Simultaneous measurement of HbAc1% and the blood glucose level with a single device enables a more rapid diagnosis of diabetes. Further, simultaneous measurement of HbA1c% with not only the blood glucose level, but also other diabetes markers such as the ratio of glycoalbumin to total albumin or the level of 1,5-anhydro-D-glucitol (also known as 1,5-AG), can contribute to understanding various pathologies including diabetes.

The blood analysis device disclosed in Patent Document 1 measures both HbAc1% and the blood glucose level using test pieces supporting an enzymatic reagent. In this method, there is one region that is measured optically to ascertain the degree of the enzyme reaction, but because a test piece for measuring HbA1c% and a second test piece for measuring the blood glucose level must be prepared, the method is complex. Further, following spot application of the blood specimen to the test piece, the test piece must be mounted manually in the measurement device, and therefore despite the fact that the reaction is an enzymatic reaction, control of the reaction temperature and reaction time is difficult, meaning issues with the measurement accuracy remain.

The blood analysis device disclosed in Patent Document 2 is constructed to enable measurement of HbA1c% and measurement of the blood glucose level to be conducted by a single sampling of a blood sample. Moreover, the HbA1c% measurement is conducted using the principles of liquid chromatography, which offers superior measurement accuracy to a measurement using a test piece. However, although the HbA1c% measurement involves conducting liquid chromatography and then conducting measurements using an absorbance meter, in the glucose measurement, an enzyme electrode method is used. Accordingly, an enzyme electrode for measuring the glucose and a detector for measuring the glycohemoglobin must be provided separately. The enzyme electrode is a consumable item, and must be replaced more frequently than the light source of the absorbance meter, making maintenance more complex. Furthermore, the enzyme electrode requires the use of a rare metal such as platinum, or silver, and therefore a complex separation operation must be conducted upon disposal. Moreover, when whole blood is used as the blood sample and the glucose is measured by the enzyme electrode method, a step of passing the blood sample through a blood cell separation membrane to remove the blood cell component and isolate the plasma component is required, which increases the complexity.

The present invention has been developed in light of these circumstances, and has an object of providing a blood analysis device and a blood analysis method that enable the analysis of at least two measurement items and use a simplified detection mechanism.

### Solution to Problem

The present invention include the aspects described below.
[1] A blood analysis device including:
   an introduction section into which a blood sample is introduced,
   a first reagent holder in which a first reagent for measuring a first measurement item of the blood sample is stored,
   a first pump that controls liquid delivery of the first reagent,
   a second reagent holder in which a second reagent for measuring a second measurement item of the blood sample is stored,
   a liquid delivery control mechanism which is connected to the introduction section, the first pump and the second reagent holder, and controls liquid delivery of the blood sample and the second reagent,
   a first line which is connected to the liquid delivery control mechanism, and into which at least a portion of the blood sample is introduced,
   a liquid chromatography column which is connected to the first line, and
   a flow cell optical detector which is connected to the liquid chromatography column and measures the first measurement item and the second measurement item.
[2] The blood analysis device according to [1], further including:
   a second line which connects the liquid delivery control mechanism and the flow cell optical detector.
[3] The blood analysis device according to [2], wherein
   the liquid delivery control mechanism contains a flow path switching unit between the introduction section and the second line,
   the second line connects the flow path switching unit and the flow cell optical detector, and
   the flow path switching unit can be switched to block the connection between the introduction section and the liquid chromatography column, and connect the introduction section and the second line.
[4] The blood analysis device according to [3], wherein the liquid delivery control mechanism also includes a second pump connected to the flow path switching unit, and an injection port positioned between the flow path switching unit and the introduction section.
[5] The blood analysis device according to any one of [1] to [4], wherein the flow cell optical detector detects absorbance.
[6] The blood analysis device according to any one of [1] to [5], further including:
   a third reagent holder in which a third reagent for measuring the first measurement item is stored,
   a third pump which controls liquid delivery of a third reagent, and
   a mixer which is connected to the first pump and the third pump, and mixes the first reagent and the third reagent, wherein
   the mixer is connected to the liquid delivery control mechanism, and
   a mixed liquid of the first reagent and the third reagent is supplied to the liquid chromatography column via the liquid delivery control mechanism.
[7] The blood analysis device according to any one of [1] to [6], further including:
   a diluent holder in which a reagent for diluting the blood sample is stored, wherein
   the diluent holder is connected to the introduction section via the liquid delivery control mechanism.
[8] The blood analysis device according to any one of [1] to [7], further including a first temperature adjustment mechanism for adjusting the temperature of at least the flow cell optical detector.
[9] The blood analysis device according to [8], further including a second temperature adjustment mechanism for adjusting the temperature of at least one of the first reagent holder and the second reagent holder.
[10] The blood analysis device according to any one of [1] to [9], further including:
   a fourth reagent holder in which a fourth reagent for measuring the second measurement item is stored, wherein
   the fourth holder is connected to the liquid delivery control mechanism, and
   the fourth reagent is introduced into the introduction section from the fourth reagent holder via the liquid delivery control mechanism.
[11] The blood analysis device according to any one of [1] to [10], wherein the first measurement item is HbA1c%.
[12] The blood analysis device according to any one of [1] to [11], wherein the second measurement item is glucose concentration.
[13] A blood analysis method including:
   a step of introducing a portion of a blood sample and a first reagent into a liquid chromatography column,
   a step of separating a first component of the blood sample in the liquid chromatography column,
   a step of measuring the concentration of the first component using a flow cell optical detector, and
   a step of measuring the concentration of a second component in the blood sample using the flow cell optical detector, using the remainder of the blood sample and at least a second reagent.
[14] The blood analysis method according to [13], wherein the step of measuring the concentration of the second component in the blood sample using the flow cell optical detector includes a step of preparing a mixed liquid by adding the second reagent to the remainder of the blood sample.
[15] The blood analysis method according to [13] or [14], wherein the step of preparing the mixed liquid is conducted either during or after the step of separating the first component.
[16] The blood analysis method according to any one of [13] to [15], wherein the step of measuring the concentration of the second component using the flow cell optical detector is conducted after the step of measuring the first component.
[17] The blood analysis method according to any one of [13] to [16], wherein the first component is HbA1c.
[18] The blood analysis method according to any one of [13] to [17], wherein the second component is glucose.
[19] The blood analysis method according to [18], further including:
   a step of calculating total hemoglobin from the chromatogram of the blood sample separated in the liquid chromatography column using the flow cell optical detector, and
   a step of correcting the glucose concentration based on the total hemoglobin.
[20] The blood analysis method according to any one of [13] to [19], wherein the blood sample is whole blood.
[21] The blood analysis method according to any one of [13] to [20], wherein the step of measuring the concentration of the second component uses the measurement principles of the enzyme method.
[22] The blood analysis method according to [21], wherein the step of measuring the concentration of the second component includes a step of measuring the amount of hydrogen peroxide generated by the reaction of the second component and an enzyme.
[23] The blood analysis method according to [22], wherein a Trinder's reagent is used in the step of measuring the concentration of the second component.
[24] The blood analysis method according to [23], wherein the Trinder's reagent is a reagent having a color development peak within a range from 580 to 900 nm upon reaction with hydrogen peroxide.
[25] The blood analysis method according to [23] or [24], wherein the Trinder's reagent contains MAOS (CAS number [82692-97-5]).
[26] The blood analysis method according to any one of [23] to [25], wherein the Trinder's reagent is incorporated in the second reagent.
[27] The blood analysis method according to any one of [13] to [26], wherein the step of introducing a portion of the blood sample and the first reagent into a liquid chromatography column includes a step of diluting the blood sample with a diluent.
[28] The blood analysis method according to [27], wherein the step of introducing a portion of the blood sample and the first reagent into a liquid chromatography column includes a step of diluting the blood sample with a diluent, and
   the diluent contains at least one component selected from the group consisting of one or more enzymes used in the enzyme method reaction, color developers, and coupling agents.
[29] The blood analysis method according to [28], wherein the diluent contains a coupling agent or a color developer.
[30] The blood analysis method according to any one of [13] to [29], wherein in the step of measuring the concentration of the second component in the blood sample using the flow cell optical detector, a fourth reagent is also used.
[31] The blood analysis method according to [30], wherein the step of measuring the concentration of the second component uses the measurement principles of the enzyme method, and the fourth reagent contains at least one component selected from the group consisting of one or more enzymes used in the enzyme method reaction, color developers, and coupling agents.

### Advantageous Effects of Invention

The aspects described above are able to provide a blood analysis device and a blood analysis method that employ a simplified detection mechanism.

### Brief Description of the Drawings

FIG. 1 is a schematic diagram of one aspect of the blood analysis device according to one embodiment.
FIG. 2 is a schematic diagram of one aspect of the blood analysis device according to another embodiment.
FIG. 3 is a schematic diagram of one aspect of the blood analysis device according to another embodiment.
FIG. 4 is a schematic diagram of one aspect of the blood analysis device according to another embodiment.
FIG. 5 is a schematic diagram of one aspect of the blood analysis device according to another embodiment.
FIG. 6 is a schematic diagram of one aspect of the blood analysis device according to another embodiment.
FIG. 7 is a schematic diagram of one aspect of the blood analysis device according to another embodiment.
FIG. 8 is a schematic diagram of one aspect of the blood analysis device according to another embodiment.
FIG. 9 is a schematic diagram of one aspect of the blood analysis device according to another embodiment.
FIG. 10 is a graph illustrating the OD value at 505 nm plotted against the measurement time during measurement of glucose aqueous solutions B.
FIG. 11 is a graph illustrating the integral of the OD value at 505 nm plotted against the glucose concentration during measurement of glucose aqueous solutions B.
FIG. 12 is a graph illustrating the OD value at 505 nm plotted against the measurement time during measurement of glucose-added whole blood samples.
FIG. 13 is a graph illustrating the integral of the OD value at 505 nm plotted against the glucose concentration during measurement of glucose-added whole blood samples.
FIG. 14 is an absorption spectrum of whole blood diluted 101-fold with a hemolysis washing solution.
FIG. 15 is a graph illustrating the OD value at 630 nm plotted against the measurement time during measurement of whole blood samples.
FIG. 16 is a graph illustrating the integral of the OD value at 630 nm plotted against the glucose concentration of whole blood samples.
FIG. 17 is a graph illustrating the OD value at 660 nm plotted against the measurement time during measurement of whole blood samples.
FIG. 18 is a graph illustrating the integral of the OD value at 660 nm plotted against the glucose concentration of whole blood samples.
FIG. 19 illustrates the results of comparing (a) the HbA1c elution peak and (b) the HbA0 elution peak when a whole blood sample is diluted with a diluent containing a known hemolysis washing solution for RC20 or 4-aminoantipyrine.

### Description of Embodiments

One aspect of the present invention is described below with reference to the drawings.

In this description, glycohemoglobin refers to one of hemoglobin A1, hemoglobin A1a, hemoglobin A1b and hemoglobin A1c. From the viewpoint of being advantageous in determining the blood glucose control status, the more stable hemoglobin A1c (hereinafter abbreviated as HbA1c) is preferable.

In this description, "HbA1c%" means the percentage of HbA1c relative to total hemoglobin in a blood sample.

In this description, the "glucose concentration" means the mass of glucose relative to the volume of blood plasma (units: mg/dL) contained in a blood sample.

### <Blood Analysis Device>

A blood analysis device in one aspect of the present invention includes an introduction section, a first reagent holder, a first pump, a second reagent holder, a liquid delivery control mechanism, a first line, a liquid chromatography column, and a flow cell optical detector. A blood sample is introduced into the introduction section. The first reagent holder stores a first reagent for measuring a first measurement item of the blood sample. The pump controls liquid delivery of the first reagent. The second reagent holder stores a second reagent for measuring a second measurement item of the blood sample. The liquid delivery control mechanism is connected to the introduction section, the first pump and the second reagent holder, and controls liquid delivery of the blood sample and the second reagent. The first line is connected to the liquid delivery control mechanism, and at least a portion of the blood sample is introduced into the first line. The liquid chromatography column is connected to the first line. The flow cell optical detector is connected to the liquid chromatography column and measures the first measurement item and the second measurement item.

In this description, an expression that "A is connected to B" includes the case in which A is connected directly to B, and the case in which is A is connected indirectly to B. The expression that "A is connected indirectly to B" means that A is connected to B via C. For example, a description that "the flow cell optical detector is connected to the liquid chromatography column" includes the case in which the flow cell optical detector is connected to the liquid chromatography column via a line or the like. In another aspect, in this description, the expression that "A is connected to B" means a state in which a liquid such as a sample can flow between A and B.

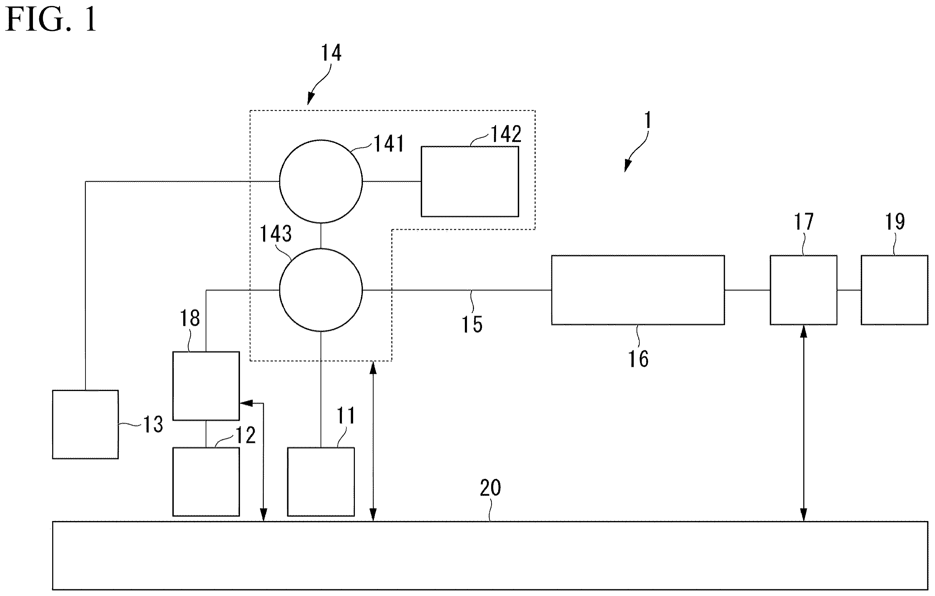

FIG. 1 is a schematic diagram of one aspect of the blood analysis device according to an embodiment of the present invention. The blood analysis device 1 illustrated in FIG. 1 includes an introduction section 11, a first reagent holder 12, a second reagent holder 13, a liquid delivery control mechanism 14, a first line 15, a liquid chromatography column 16, a flow cell optical detector 17, a pump 18, a waste liquid tank 19, and a control unit 20. The liquid delivery control mechanism 14 has a first flow path switching unit 141, a pump 142 and an injection port 143. The introduction section 11 is connected to the injection port 143 of the liquid delivery control mechanism 14. The first reagent holder 12 is connected to the injection port 143 via the pump 18. The second reagent holder 13 is connected to the first flow path switching unit 141. The first line 15 connects the injection port 143 and the liquid chromatography column 16. The liquid chromatography column 16 is connected to the flow cell optical detector 17. The waste liquid tank 19 is connected to the flow cell optical detector 17. The control unit 20 controls the liquid delivery control mechanism 14, the flow cell optical detector 17 and the pump 18.

This embodiment describes an example in which the first measurement item is HbA1c%, and the second measurement item is the glucose concentration.

The introduction section 11 is the section into which a sample containing a blood-derived component (hereinafter also referred to as a "blood sample") is introduced. The blood sample may be blood, or a sample prepared by diluting blood. In those cases where the blood sample is blood, a sample cup, a blood collection tube, or a blood sample collected using a lancet may be set at the introduction section 11. Further, a needle such as a sample probe may be used as the introduction section 11, or an injection system using a sample loop may be used. In those cases where a plurality of specimens are to be processed in a continuous manner, the introduction section 11 may be an autosampler or the like containing the type of injection port 143 described below.

The first reagent holder 12 stores the first reagent for measuring the first measurement item. In this embodiment, the first measurement item is the HbA1c%, and an eluent for the liquid chromatography column is stored as the first reagent. The pump 18, which is used for aspirating the first reagent from the first reagent holder 12, is attached to the first reagent holder 12. A degassing device (also termed a "degasser") may be provided either between the first reagent holder 12 and the pump 18, or between the pump 18 and the liquid delivery control mechanism 14.

A composition that is suitable for eluting hemoglobin may be selected as the eluent. Examples of the eluent include conventional buffer solutions and organic solvents including salt compounds. Specific examples of the eluent include organic acids such as citric acid, succinic acid, tartaric acid and malic acid, as well as salts of these acids, amino acids such as glycine, taurine and arginine, inorganic acids such as hydrochloric acid, nitric acid, sulfuric acid, phosphoric acid, boric acid and acetic acid, as well as salts of these acids, and buffer solutions such as Good's buffers. The above buffer solutions may also contain suitable amounts of typically added substances such as surfactants, various polymers and hydrophilic low-molecular weight compounds.

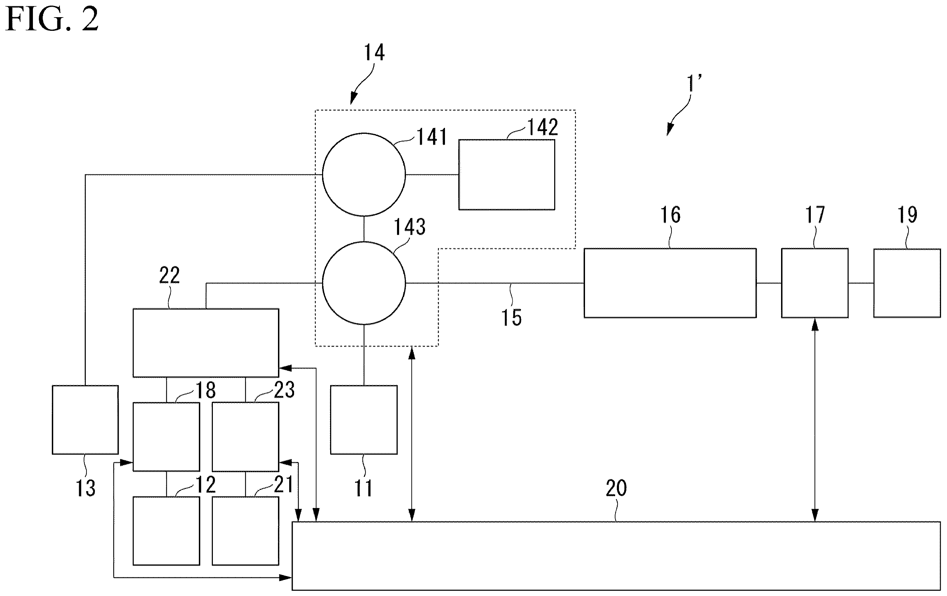

Further, eluents having different pH levels or salt concentrations may be used as the eluent. The number of different eluents may be two or three. In such cases, as illustrated in FIG. 2, the blood analysis device 1' may also have a third reagent holder 21 in which a third reagent having a different pH or salt concentration from the first reagent is stored, a mixer 22 to enable elution of a gradient, and a pump 23 for aspirating the third reagent from the third reagent holder 21. A degassing device may be provided either between the third reagent holder 21 and the pump 23, or between the pump 23 and the mixer 22. Furthermore, in the blood analysis device 1' illustrated in FIG. 2, the control unit 20 controls the operations of the liquid delivery control mechanism 14, the flow cell optical detector 17, the pump 18, the mixer 22 and the pump 23.

The second reagent holder 13 stores the second reagent for measuring the second measurement item. In this embodiment, the second measurement item is the glucose concentration, and details regarding the second reagent are described below in detail in the section entitled <Blood Analysis Method>.

The liquid delivery control mechanism 14 is connected to the introduction section 11, the pump 18 and the second reagent holder 13. The control unit 20 controls the operation of the liquid delivery control mechanism 14, thereby controlling the liquid delivery of the blood sample and the second reagent. The liquid delivery control mechanism 14 preferably has the first flow path switching unit 141 and the pump 142 for controlling the liquid delivery of the blood sample and the second reagent. Moreover, the liquid delivery control mechanism 14 preferably also has the injection port 143 for delivering the blood sample, the first reagent and the second reagent to the first line 15. Examples of the first flow path switching unit 141 include an autovalve or an electromagnetic valve. An example of the pump 142 is a syringe pump. A conventional injection port may be used as the injection port 143. Further, the liquid delivery control mechanism 14 may also include a degassing device. Furthermore, the liquid delivery control mechanism 14 may also include a mechanism for controlling the pressure of the entire liquid chromatography column 16 described below.

The control unit 20 operates the first flow path switching unit 141 so as to link the introduction section 11 and the pump 142, and uses the pump 142 to aspirate a portion of the blood sample. The aspirated blood sample may be stored inside the pump 142, or a separate section for storing solutions may be provided between the introduction section 11 and the pump 142, with the blood sample stored in that separate section. The section for storing solutions may be a sample loop, or may be the same type of component as the first line.

The first reagent is aspirated from the first reagent holder 12 by the pump 18, and is introduced continuously into the first line 15 and the liquid chromatography column 16 via the injection port 143. While the first reagent is being introduced into the first line and the liquid chromatography column 16, the aspirated blood sample is introduced into the first line 15 and the liquid chromatography column 16 via the injection port 143.

Next, the control unit 20 switches the first flow path switching unit 141 so as to link the second reagent holder 13 and the pump 142 , and uses the pump 142 to aspirate the second reagent from the second reagent holder 13. At this time, a separate section for storing solutions may be provided between the first flow path switching unit 141 and the pump 142, with the second reagent being stored in that separate section. This section for storing solutions may be a sample loop, or may be the same type of component as the first line.

Subsequently, the control unit 20 links the first flow path switching unit 141 to the introduction section 11, and the aspirated second reagent is injected into the introduction section 11 by the pump 142. A needle such as a sample probe may be used during this injection of the second reagent into the introduction section 11. Following injection of the second reagent into the introduction section 11, aspiration and discharge from the needle may be repeated to mix the blood sample and the second reagent. The mixing of the blood sample and the second reagent may be conducted by agitation using an ultrasonic generator or the like.

The mixed liquid of the blood sample and the second reagent is introduced into the first line 15 via the injection port 143. In this embodiment, as described above, a portion of the blood sample and the first reagent is introduced into the liquid chromatography column 16 via the first line 15. Subsequently, a mixed liquid of the remainder of the blood sample and the second reagent is introduced into the liquid chromatography column 16 via the first line 15. The liquid chromatography column 16 separates the HbA1c in the blood sample from other blood components, specifically hemoglobin other than HbA1c.

The liquid chromatography column 16 is a column filled with a solid phase having a cation exchange capability. For example, a column or the like filled with a resin to which cation exchange groups are bonded may be used as the liquid chromatography column 16. Examples of these cation exchange groups include carboxyl groups, phosphate groups, and sulfonate groups. Among these, sulfonate groups are preferred.

There are no particular limitations on the filler particles of the solid phase having the cation exchange capability, and any filler particles typically used for ion exchange chromatography may be used, including inorganic-based particles and organic-based particles. Examples of inorganic-based particles include particles formed from silica or zirconia or the like. Examples of the organic-based particles include natural polymer particles such as cellulose, polyamino acids and chitosan, and synthetic polymer particles such as polystyrene and polyacrylate esters. Examples of preferred solid phases having cation exchange groups include the fillers disclosed in Japanese Unexamined Patent Application, First Publication No. 2011-047858, containing crosslinked polymer particles obtained by polymerizing a mixture of a non-crosslinkable hydrophilic acrylic monomer and polyglycidyl ethers, and a layer formed by polymerizing an acrylic-based monomer layer having a cation exchange group on the surface of the crosslinked polymer particles.

The liquid chromatography column 16 may be housed inside a temperature control mechanism such as a column oven or the like.

The flow cell optical detector 17 is connected to the liquid chromatography column 16. After passage of the blood sample through the liquid chromatography column 16, the first and second measurement items in the blood sample are measured by the flow cell optical detector 17. In this embodiment, the flow cell optical detector 17 detects glycohemoglobin and other blood components that have been separated in the liquid chromatography column 16, and produces chromatogram data. Using the thus obtained chromatogram data, the HbA1c% can be measured. Further, the flow cell optical detector 17 also detects the glucose concentration contained in the mixed liquid of the blood sample and the second reagent.

An absorbance meter installed as the detector of a high-performance liquid chromatography (also abbreviated as HPLC) device may be used as the flow cell optical detector 17. In other words, the flow cell optical detector 17 may detect absorbance. The flow cell optical detector 17 may also be a device for measuring scattered light, fluorescence or chemiluminescence.

Following passage through the flow cell optical detector 17, the first reagent, the second reagent and the blood sample are discharged into the waste liquid tank 19.

The control unit 20 controls the aforementioned operations of the liquid delivery control mechanism 14, the flow cell optical detector 17 and the pump 18. The control unit 20 is constructed using an information processing device. In other words, the control unit 20 contains a CPU (Central Processor Unit), memory and an auxiliary storage device connected via a bus. The control unit 20 is operated by executing a program. The control unit 20 may be realized using a computer. In such cases, a program for executing the control functions may be recorded on a computer-readable storage medium, and this program stored on the storage medium then read into the computer system and executed in order to carry out the control functions.

Here, the term "computer system" refers to a computer system housed within the blood analysis device, including an OS and hardware such as peripheral equipment. Further, the term "computer-readable storage medium" may refer to a portable medium such as a flexible disk, magneto-optical disk, ROM or CD-ROM, or to a storage device such as a hard disk housed within the computer system. The "computer-readable storage medium" may also include devices which hold the program for a short period while in transit, such as communication lines used for transmitting the program via networks such as the internet or communication lines such as telephone lines, or devices which hold the program for a fixed period such as the volatile memory within the computer systems that function as servers or clients during transmission of the program. Further, the program described above may be used for executing a portion of the functions described above, or may be used in combination with other programs already stored in the computer system to execute the above functions. Furthermore, a portion of the control unit of the blood analysis device in the above embodiment may also be realized using an integrated circuit such as an LSI (Large Scale Integration). The control unit of the blood analysis device may be processor controlled, or a portion or all of the control unit may be integrated and processor controlled. Further, the technique used for circuit integration is not limited to LSI, and a dedicated circuit or general-purpose processor may also be used. Furthermore, if progress in semiconductor technology leads to the development of circuit integration techniques that can replace LSI, then circuit integration using those techniques may also be used.

The blood analysis device 1 described above can measure both HbA1c% and the glucose concentration using the flow cell optical detector 17, and therefore the detection mechanism can be simplified, and the overall device can be reduced in size. Further, because only a single detection mechanism is used, maintenance is simpler.

### <Blood Analysis Device Variation 1>

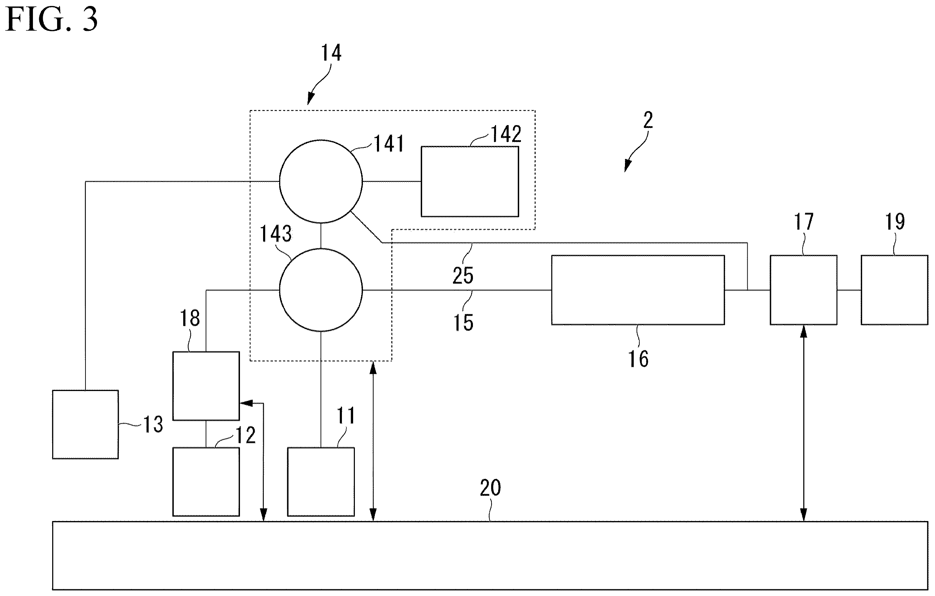

FIG. 3 is a schematic diagram of a blood analysis device according to a Variation 1 of the present invention. The blood analysis device 2 further includes a second line 25. Other structures in the blood analysis device 2 are the same as those of the blood analysis device 1, and are therefore not described here.

The second line 25 is provided between the liquid delivery control mechanism 14 and the flow cell optical detector 17. In this embodiment, the second line 25 is connected to the first flow path switching unit 141.

In the blood analysis device 2, the liquid delivery of the blood sample and the second reagent differs from the blood analysis device 1 in the manner described below. The control unit 20 operates the first flow path switching unit 141 so as to link the introduction section 11 and the pump 142, and uses the pump 142 to aspirate a portion of the blood sample. The first reagent is aspirated from the first reagent holder 12 by the pump 18, and is introduced continuously into the first line 15 and the liquid chromatography column 16 via the injection port 143. While the first reagent is being introduced into the first line and the liquid chromatography column 16, the aspirated blood sample is introduced into the first line 15 and the liquid chromatography column 16 via the injection port 143.

The first flow path switching unit 141 is then switched so as to link the second reagent holder 13 and the pump 142, and uses the pump 142 to aspirate the second reagent from the second reagent holder 13. Subsequently, the first flow path switching unit 141 is switched so as to link the pump 142 and the introduction section 11, and the aspirated second reagent is injected into the introduction section 11 and mixed with the blood sample.

The pump 142 is used to aspirate the mixed liquid of the blood sample and the second reagent, and the mixed liquid is held in the region between the first flow path switching unit 141 and the pump 142. Subsequently, the first flow path switching unit 141 is operated so as to link the pump 142 and the second line 25. The aspirated mixed liquid of the blood sample and the second reagent is then supplied to the second line 25 and introduced into the flow cell optical detector 17 by the pump 142. Following introduction of the mixed liquid into the flow cell optical detector 17, the pump 142 may be stopped for a certain period to ensure sufficient time for the reaction of the components used for measuring the blood sample and the glucose concentration. Once this prescribed reaction time has elapsed, the pump 142 may be restarted so that the mixed liquid in the flow cell optical detector 17 is discharged.

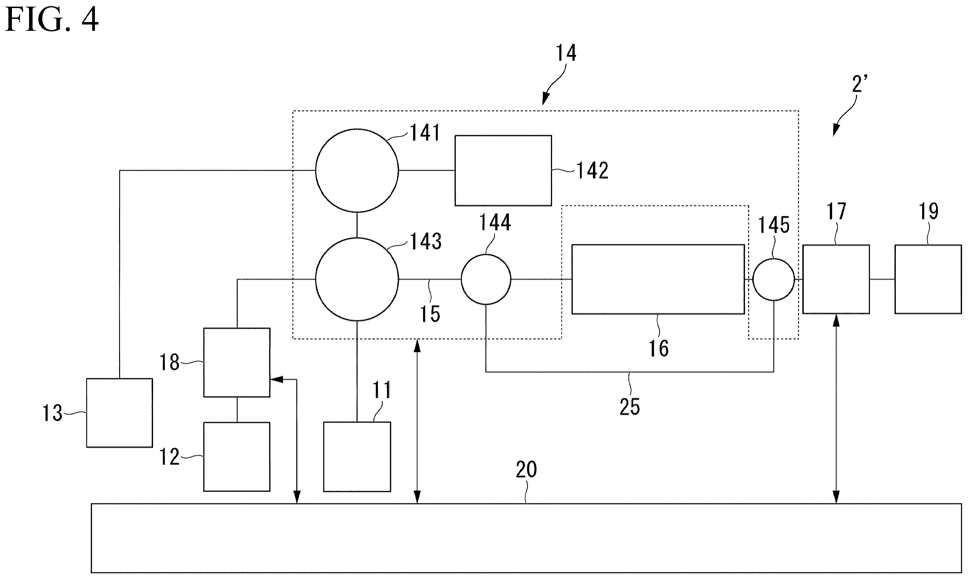

FIG. 4 is a schematic diagram of a blood analysis device according to a Variation 1' of the present invention. The blood analysis device 2' further includes a second flow path switching unit 144, a third flow path switching unit 145, and the second line 25. Other structures in the blood analysis device 2' are the same as those of the blood analysis device 1, and are therefore not described here.

The second flow path switching unit 144 and the third flow path switching unit 145 are provided in the first line 15. The second flow path switching unit 144 is connected to the third flow path switching unit 145 via the second line 25. The third flow path switching unit 145 is positioned between the liquid chromatography column 16 and the flow cell optical detector 17. Devices similar to the first flow path switching unit 141 may be used as the second flow path switching unit 144 and the third flow path switching unit 145. The third flow path switching unit 145 may also use a check valve.

In the blood analysis device 2', the liquid delivery of the blood sample and the second reagent differs from the blood analysis device 1 in the manner described below. The first flow path switching unit 141 is operated so as to link the introduction section 11 and the pump 142, and the pump 142 is used to aspirate a portion of the blood sample. Subsequently, the second flow path switching unit 144 is operated so as to link the injection port 143 and the liquid chromatography column 16, and the third flow path switching unit 145 is operated so as to link the liquid chromatography column 16 and the flow cell optical detector 17. The pump 18 is used to aspirate the first reagent from the first reagent holder 12, and the first reagent is introduced continuously into the first line 15 and the liquid chromatography column 16 via the injection port 143. While the first reagent is being introduced into the first line 15 and the liquid chromatography column 16, the aspirated blood sample is introduced into the first line 15 and the liquid chromatography column 16 via the injection port 143.

The first flow path switching unit 141 is then switched so as to link the second reagent holder 13 and the pump 142, and the pump 142 is used to aspirate the second reagent from the second reagent holder 13. Subsequently, the first flow path switching unit 141 is switched so as to link the pump 142 and the introduction section 11, and the aspirated second reagent is injected into the introduction section 11 and mixed with the blood sample.

The second flow path switching unit 144 blocks passage between the first line 15 and the liquid chromatography column 16, and links the first line 15 and the second line 25. The third flow path switching unit 145 blocks passage between the liquid chromatography column 16 and the flow cell optical detector 17, and links the second line 25 and the flow cell optical detector 17. Using the pump 142, the mixed liquid of the blood sample and the second reagent is introduced into the flow cell optical detector 17 via the injection port 143, the second flow path switching unit 144, the second line 25 and the third flow path switching unit 145. In the blood analysis device 2', the second line 25 is linked to the flow cell optical detector 17 via the third flow path switching unit 145, but the present invention is not limited to this configuration, and for example, the second line may be joined directly to the flow cell optical detector 17.

In Variation 1 and Variation 1' of the present invention, the mixture of the blood sample and the second reagent is not introduced into the liquid chromatography column 16, but rather passes through the second line 25 and into the flow cell optical detector 17. As a result, contamination of the liquid chromatography column 16 by the mixture of the blood sample and the second reagent can be prevented, and the measurement time can be shortened.

### <Blood Analysis Device Variation 2>

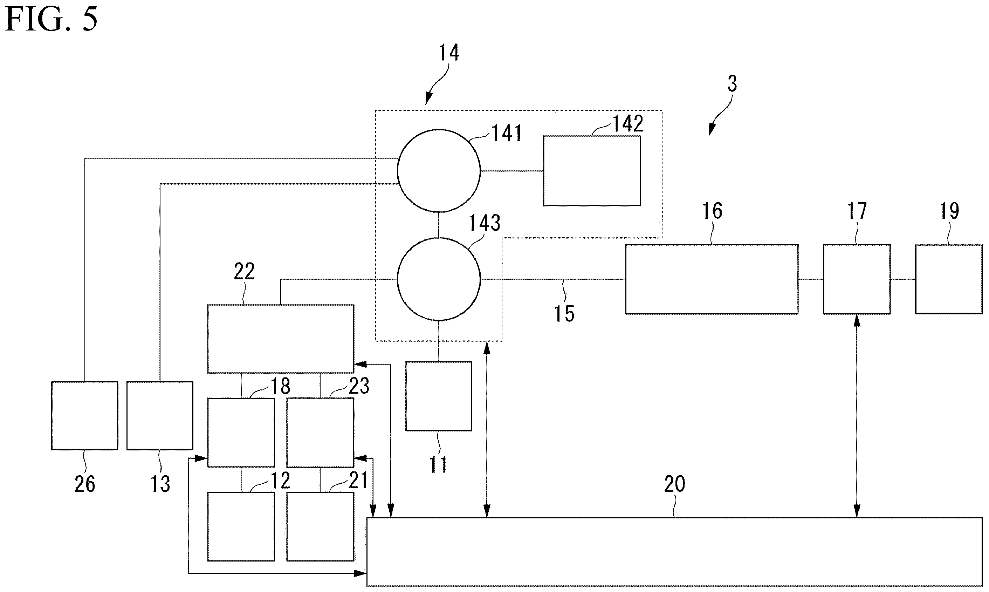

FIG. 5 illustrates a Variation 2 of the blood analysis device of the present invention. The blood analysis device 3 adds a diluent holder 26 to the blood analysis device 1' illustrated in FIG. 2. Other structures in the blood analysis device 3 are the same as those of the blood analysis device 1', and are therefore not described here.

The diluent holder 26 stores a diluent for diluting the blood. Examples of diluents that may be used include hypotonic solutions, and more specifically buffer solutions which may also contain preservatives or surfactants. In those cases where the blood sample introduced into the introduction section 11 is blood, the first flow path switching unit 141 is operated so as to connect the diluent holder 26 and the introduction section 11, and the pump 142 is used to inject the diluent from the diluent holder 26 into the introduction section 11. A needle such as a sample probe may be used when injecting the diluent into the introduction section 11. Following injection of the diluent into the introduction section 11, aspiration and discharge from the needle may be repeated to mix the blood sample and the diluent. The mixing of the blood sample and the diluent may be conducted by agitation using an ultrasonic generator or the like.

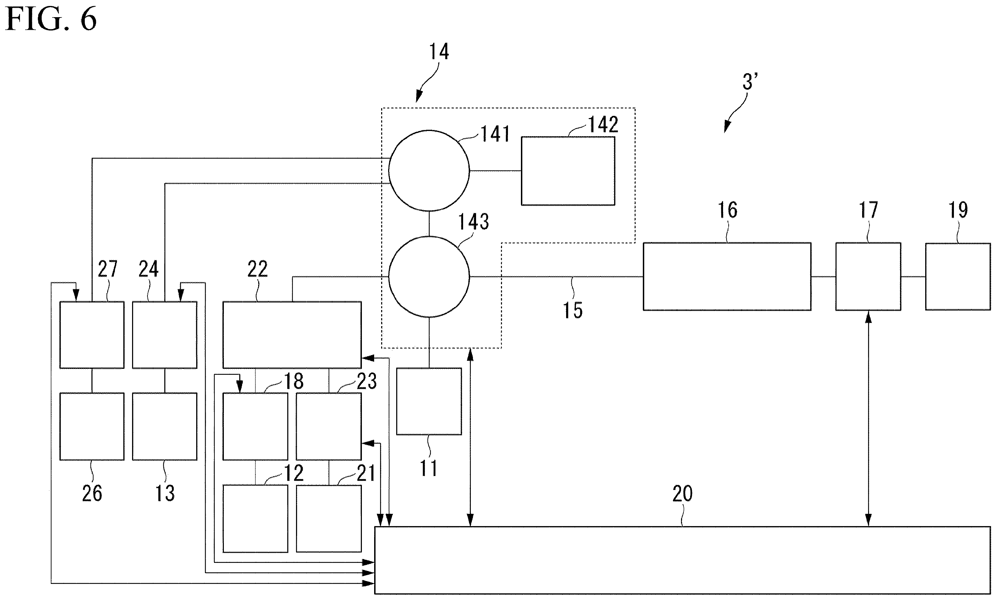

The blood analysis device 3' illustrated in FIG. 6 is a variation of the blood analysis device 3, and has a pump 24 positioned between the second reagent holder 13 and the first flow path switching unit 141. Further, the blood analysis device 3' also has a pump 27 positioned between the diluent holder 26 and the first flow path switching unit 141.

By using this variation, the blood sample can be diluted with the diluent and then introduced into the blood analysis device.

### <Blood Analysis Device Variation 3>

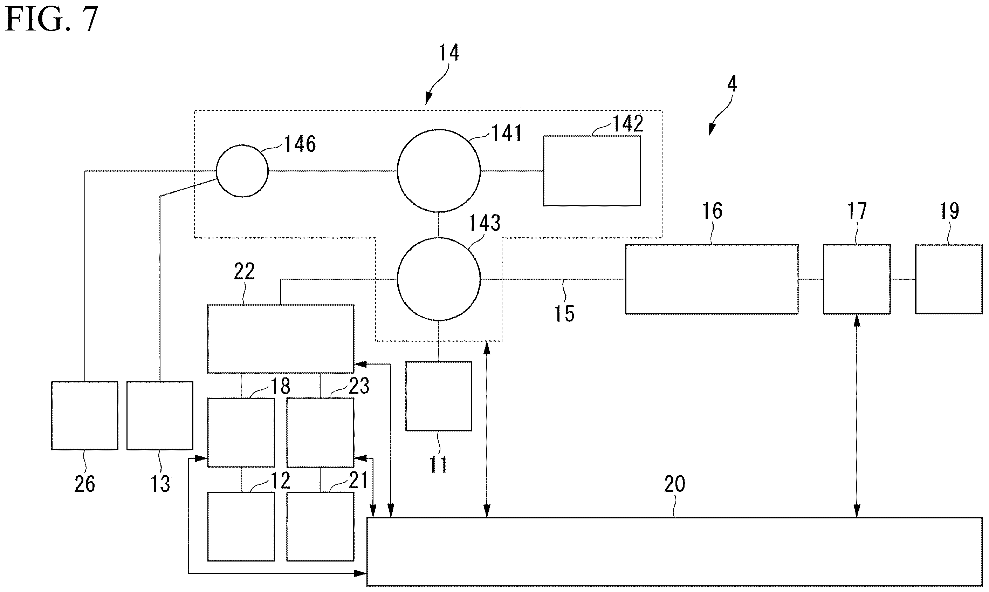

FIG. 7 illustrates a Variation 3 of the blood analysis device of the present invention. This blood analysis device 4 adds a fourth flow path switching unit 146 to the blood analysis device 3 illustrated in FIG. 5. A device similar to the first flow path switching unit 141 may be used as the fourth flow path switching unit 146. Other structures in the blood analysis device 4 are the same as those of the blood analysis device 3, and are therefore not described here. The fourth flow path switching unit 146 connects the first flow path switching unit 141 to the second reagent holder 13 or the diluent holder 26. The fourth flow path switching unit 146 and the first flow path switching unit 141 are operated so as to connect the diluent holder 26 and the introduction section 11, and the diluent is injected from the diluent holder 26 into the introduction section 11. Further, in those cases where the second reagent and the blood sample are to be mixed, the fourth flow path switching unit 146 and the first flow path switching unit 141 are operated so as to connect the second reagent holder 13 and the introduction section 11, thereby injecting the second reagent from the second reagent holder 13 into the introduction section 11.

By using this variation, the number of ports in the first flow path switching unit 141 can be limited.

### <Blood Analysis Device Variation 4>

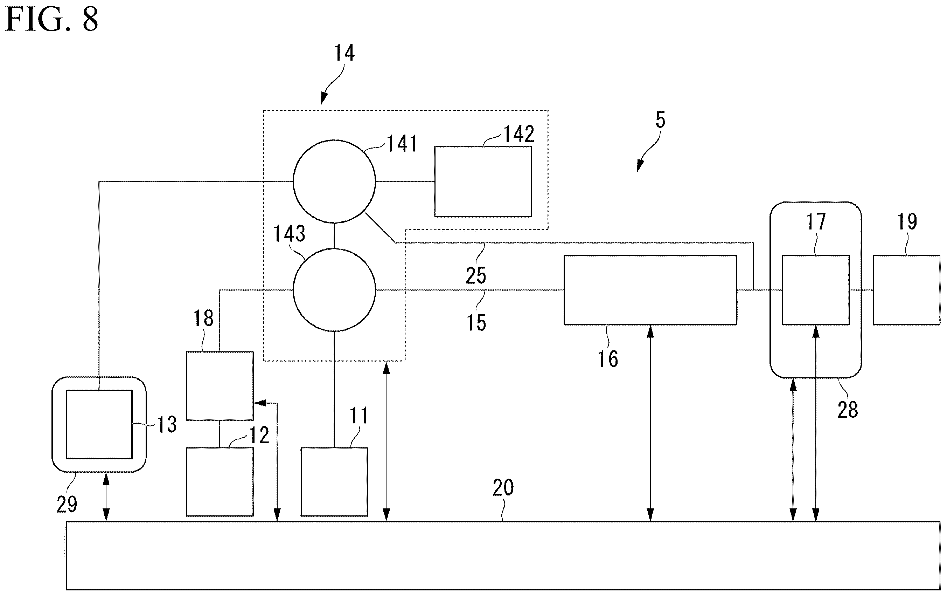

FIG. 8 illustrates a Variation 4 of the blood analysis device of the present invention. This blood analysis device 5 adds a first temperature adjustment mechanism 28 and a second temperature adjustment mechanism 29 to the blood analysis device 2 illustrated in FIG. 3. Other structures in the blood analysis device 5 are the same as those of the blood analysis device 2, and are therefore not described here.

The first temperature adjustment mechanism 28 adjusts the temperature of at least the flow cell optical detector 17 within a range from 20 to 50°C. This enables the output from the flow cell optical detector 17 to be stabilized. Further, in those cases where the second reagent is reacted with the blood sample, by maintaining the optimum temperature, the reactivity can be enhanced. Furthermore, the reaction rate between the second reagent and the blood sample becomes less likely to be affected by the environment such as the temperature of the surroundings in which the device is installed. The first temperature adjustment mechanism 28 may also be used to regulate the temperature of the liquid chromatography column 16 as well as the flow cell optical detector 17. Moreover, the first temperature adjustment mechanism 28 may also be used to regulate the temperature of one or more of the second line 25, the introduction section 11, the first line 15, and the injection port 143. The first temperature adjustment mechanism 28 may be a column oven.

The second temperature adjustment mechanism 29 adjusts the temperature of at least one of the first reagent holder 12 and the second reagent holder 13 to a temperature within a range from 0 to 30°C. This enables degradation of the components contained in each of the reagents to be suppressed, enables the separation performance of the liquid chromatography to be better maintained, and enables the reactivity between the various components and the blood sample to be better maintained. It is particularly desirable that the second temperature adjustment mechanism 29 is used to regulate the temperature of the second reagent holder 13 which contains a reagent that includes an enzyme. The second temperature adjustment mechanism 29 may also be used to regulate the temperature of the diluent holder 26. FIG. 8 illustrates an example in which the second temperature adjustment mechanism 29 is used to regulate the temperature of the second reagent holder 13.

The above description discloses an example of a blood analysis device 5 containing both the first temperature adjustment mechanism 28 and the second temperature adjustment mechanism 29, but the present invention is not limited to this configuration. The blood analysis device of the present invention may have at least one of the first temperature adjustment mechanism 28 and the second temperature adjustment mechanism 29.

### <Blood Analysis Device Variation 5>

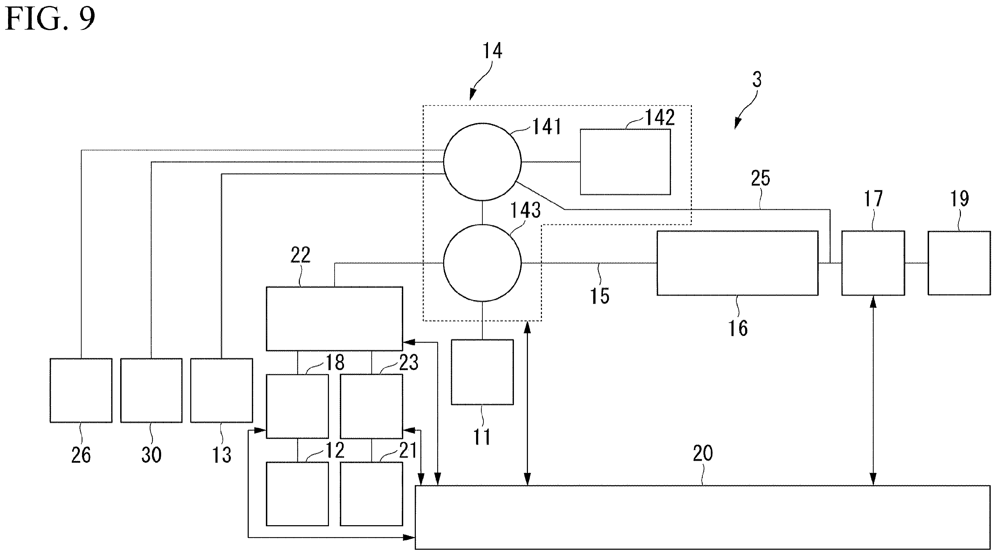

FIG. 9 illustrates a Variation 5 of the blood analysis device of the present invention. This blood analysis device 6 adds the second line 25 and a fourth reagent holder 30 to the blood analysis device 3 illustrated in FIG. 5. The second line 25 is the same as that of the blood analysis device 2 illustrated in FIG. 3, and is therefore not described here. Other structures in the blood analysis device 6 are the same as those of the blood analysis device 3, and are therefore also not described here.

The fourth reagent holder 30 stores a fourth reagent used for measuring the second measurement item of the blood sample. In other words, in this variation, the reagent for measuring the second measurement item is divided and stored in the second reagent holder 13 and the fourth reagent holder 30. The fourth reagent holder 30 is connected to the first flow path switching unit 141 of the liquid delivery control mechanism 14 in a similar manner to the second reagent holder 13.

Operation of the blood analysis device 6 is described below. The operations up to and including injection of the second reagent from the second reagent holder 13 into the introduction section 11, and mixing of the blood sample and the second reagent are the same as the operations described above in the section entitled <Blood Analysis Device>.

Subsequently, the fourth reagent is introduced, via the liquid delivery control mechanism 14, from the fourth reagent holder into the introduction section 11. More specifically, the first flow path switching unit 141 of the liquid delivery control mechanism 14 is switched so as to link the fourth reagent holder 30 and the pump 142, and the pump 142 is used to aspirate the fourth reagent from the fourth reagent holder 30. The first flow path switching unit 141 is then switched so as to link the pump 142 and the introduction section 11, and the aspirated fourth reagent is injected into the introduction section 11 and mixed with the mixed liquid containing the blood sample and the second reagent.

The pump 142 is then used to aspirate the mixed liquid of the blood sample, the second reagent and the fourth reagent, with the mixed liquid being held between the first flow path switching unit 141 and the pump 142. Subsequently, the first flow path switching unit 141 is operated so as to link the pump 142 and the second line 25. The mixed liquid of the blood sample, the second reagent and the fourth reagent is then introduced into the flow cell optical detector 17 through the second line 25.

The above description discloses an example in which the blood analysis device 6 was used as the blood analysis device containing the fourth reagent holder, but the present invention is not limited to this configuration. The blood analysis device may have a configuration in which the fourth reagent holder is added to any one of the blood analysis devices 1, 2, 4 and 5.

Moreover, the blood analysis device may include a combination of at least two of the above blood analysis devices 1 to 6. For example, Variations 1 and 2 may be combined, Variations 1, 2 and 4 may be combined, or Variations 2, 4 and 5 may be combined.

### <Other Configurations>

The blood analysis device of the present invention may also include other optional structures required for operation of the device. For example, the blood analysis device may also include a washing liquid tank that stores a washing liquid used for washing the introduction section 11 and/or the injection port 143. There are no particular limitations on the position of the washing liquid tank, provided the introduction section 11 and/or the injection port 143 can be washed. The washing liquid stored in the washing liquid tank may be aspirated using the pump 142, or a dedicated washing liquid pump may be provided. The blood analysis device may also include a separate waste liquid tank for collecting the waste liquid following washing, or may include a flow path that enables the waste liquid to be collected in the waste liquid tank 19.

The washing liquid stored in the washing liquid tank may employ a liquid containing at least one washing component such as a surfactant. Further, the washing liquid may contain an antiseptic such as 2-phenoxyethanol or sodium azide, or may contain both a surfactant and an antiseptic.

In another aspect, the blood analysis device may include a washing liquid tank that stores a washing liquid used for washing the flow cell optical detector 17. This washing liquid tank may be positioned so that the washing liquid can be supplied from a location between the liquid chromatography column 16 and the flow cell optical detector 17. A separate pump may be included for delivering the washing liquid. The blood analysis device may include a separate waste liquid tank for collecting the waste liquid following washing, or may include a flow path that enables the waste liquid to be collected in the waste liquid tank 19.

In addition, the blood analysis device may also include, for example, channels for removing air from the pumps and/or flow paths (also known as purging). The flow paths may also include backflow prevention valves (also known as self-closing valves). The blood analysis device may also include a pump for discharging liquids from the flow paths.

These other configurations may be combined with any of the blood analysis devices 1, 1', 2, 2', 3, 3', 4, 5 and 6.

### <Measurement Items>

In the aspects described above, an example was described in which the first measurement item was HbA1c% and the second measurement item was the glucose concentration, but the present invention is not limited to these measurement items. For example, the first measurement item may be HbA1c% and the second measurement item may be glycoalbumin %. In this case, the first reagent holder and the second reagent holder are used to store a reagent for measuring HbA1c% and a reagent for measuring glycoalbumin % respectively. The glycoalbumin % means the proportion of glycoalbumin relative to total albumin.

The device configuration for the portion used for measuring HbA1c% may be the same as the example above in which the first measurement item was HbA1c% and the second measurement item was the glucose concentration.

Measurement of glycoalbumin % as the second measurement item is conducted by measuring both the total albumin and glycoalbumin, and then calculating the percentage of glycoalbumin within the total albumin. The total albumin and glycoalbumin can be measured using the combinations of measurement principles shown in Table 1. In Table 1, HPLC indicates high-performance liquid chromatography.

**[Table 1]**

| Combination of measurement principles | 1 | 2 | 3 | 4 |
|---|---|---|---|---|
| Total albumin | HPLC | biochemical method | biochemical method | HPLC |
| Glycoalbumin | HPLC | enzyme method | HPLC | enzyme method |

In the case of the measurement principles combination 1, the albumin and the glycoalbumin may be separated using the liquid chromatography column 16, or may be separated using a second liquid chromatography column connected to a separately provided line. The second liquid chromatography column may be installed partway along the second line 25 illustrated in the blood analysis device 2' of Variation 1'. The second liquid chromatography column is connected to the flow cell optical detector 17, and the separated albumin and glycoalbumin are detected by the flow cell optical detector 17. An eluent for measuring the glycoalbumin is stored as the reagent for measuring glycoalbumin. This eluent may be a combination of two or more eluents.

In the case of the measurement principles combination 2, for example, a liquid reagent for measuring total albumin and a liquid reagent for measuring glycoalbumin may both be stored in the second reagent holder 13. One pump may then be provided for aspirating each reagent. The total albumin is measured using a biochemical method reagent, and the glycoalbumin is measured using an enzyme method reagent.

One example of the measurement procedure in the case of the measurement principles combination 2 is described below using the blood analysis device 2 illustrated in FIG. 3. First, in order to measure HbA1c%, a portion of the blood sample set in the introduction section 11 and the eluent that functions as the first reagent are introduced into the liquid chromatography column 16 using the operations described above. The reagent for measuring total albumin is then added from the second reagent holder 13 to the remaining blood sample in the introduction section 11. A portion of the resulting mixed liquid of the blood sample and the reagent for measuring total albumin is aspirated using the pump 142 and passed through the second line 25 to reach the flow cell optical detector 17, where the total albumin is detected. The reagent for measuring glycoalbumin is then added from a separate reagent holder provided in parallel with the second reagent holder 13 to the residual blood sample still in the introduction section 11. A portion of the resulting mixture is then aspirated and passed through the second line 25 to reach the flow cell optical detector 17, where the glycoalbumin is detected. The order in which the total albumin is detected and the glycoalbumin is detected may be reversed. Alternatively, following introduction of a portion of the blood sample into the liquid chromatography column 16 to measure HbA1c%, a portion of the blood sample remaining in the introduction section 11 may be transferred to a separately installed sample cup, with the reagent for measuring total albumin or the reagent for measuring glycoalbumin then being added to this sample cup.

In the case of the measurement principles combinations 3 and 4, the HPLC, biochemical method and enzyme method described above in the combinations 1 and 2 may be combined as appropriate. In the measurement principles combinations 3 and 4, a blood analysis device may be used in which a third line connecting the first flow path switching unit 141 and the flow cell optical detector 17 is added to the blood analysis device having a second liquid chromatography column described above in relation to the measurement principle combination 1.

Moreover, in another example, the first measurement item may be HbA1c%, and the second measurement item may be 1,5-AG. In this case, a reagent for measuring HbA1c% and a reagent for measuring 1,5-AG are stored in the first reagent holder 12 and the second reagent holder 13 respectively. With the exception that the reagent stored in the second reagent holder is a reagent for measuring 1,5-AG, the remaining configuration may be the same as that described in the example in which the second measurement item was the glucose concentration.

### <Blood Analysis Method>

The blood analysis method according to one aspect of the present invention includes a step of introducing a portion of a blood sample and a first reagent into a liquid chromatography column, a step of separating a first component of the blood sample in the liquid chromatography column, a step of measuring the concentration of the first component using a flow cell optical detector, and a step of measuring the concentration of a second component in the blood sample using the flow cell optical detector, using the remainder of the blood sample and at least a second reagent. The blood analysis method may also include a step of preparing a mixed liquid by adding at least the second reagent to the remainder of the blood sample when measuring the concentration of the second component in the blood sample using the flow cell optical detector.

The blood analysis method according to one aspect of the present invention is described below with reference to the various components of the blood analysis device 1. This embodiment describes an example in which the first measurement item is HbA1c%, and the second measurement item is the glucose concentration.

The blood sample is introduced into the introduction section 11. The blood sample may be blood, or may be a blood sample that has already undergone hemolysis or dilution. In the case where the blood sample has not been subjected to hemolysis or dilution, the blood analysis device having a diluent tank described in Variation 2 may be used to subject the blood sample to hemolysis or dilution. For example, in those cases where the blood sample is whole blood, the sample is typically diluted 50-fold to 200-fold. A hypotonic solution, or more specifically a buffer solution that may also contain preservatives or surfactants, may be used for the dilution.

The first flow path switching unit 141 is switched so as to link the introduction section 11 and the pump 142. A portion of the blood sample is aspirated by the pump 142 and, together with the first reagent, is introduced into the liquid chromatography column 16 via the first line 15. In the liquid chromatography column 16, the HbA1c in the blood sample is separated from other blood components, specifically the various types of hemoglobin other than HbA1c.

Following introduction of a portion of the blood sample and the first reagent into the liquid chromatography column 16, the liquid delivery control mechanism 14 is used to add the second reagent from the second reagent holder 13 to the remaining blood sample in the introduction section 11 using the pump 142, thus preparing a mixed liquid of the blood sample and the second reagent. This preparation of the mixed liquid is preferably conducted either while the portion of the blood sample is introduced into the liquid chromatography column 16 and the HbA1c is separated, or following this separation process. For example, the amount of the blood sample to be introduced into the liquid chromatography column 16 may be set in advance, and the pump 142 then controlled so that once the prescribed amount of the blood sample has been discharged from the introduction section 11, the discharge is halted and the second reagent is added from the second reagent holder 13 to the introduction section 11.

The second reagent may contain a reagent that functions under conventional measurement principles. Examples of the measurement method include an enzyme method, an immunoturbidimetry assay, a latex turbidometric immunoassay (also abbreviated as LTIA), a chemiluminescence immunoassay, and an electrochemiluminescence immunoassay. In those cases where a plurality of components are used in one of these measurement principles, the second reagent may contain at least one of the plurality of components.

In the case of an enzyme method, the measurement target substance contained in the blood sample is reacted with a specific enzyme, and the coloring of the reaction product or a substance derived from the reaction product is measured.

In the case where the measurement target substance is glucose, measurement principles employing an enzyme method can be broadly classified into two types. The first type is a method in which glucose is reacted with a specific enzyme to generate hydrogen peroxide, action between the generated hydrogen peroxide and peroxidase (also abbreviated as POD) causes a color developer that has been added separately to the reagent to generate a color, and the change in absorbance is then measured. Examples of this type of method include the glucose oxidase (GOD) - peroxidase method (also known as GOD-POD), the mutarotase-GOD-POD method in which mutarotase is also added to the GOD-POD method, and the pyranose oxidase-POD method. In other words, under the first measurement principle, at least an enzyme, POD and a color developer are required, and these components are included in the reagent added to the blood sample when measuring the second measurement item, namely the reagent containing the second reagent and at least one of an eluent (the first reagent or the third reagent) and a diluent. In those cases where the first reagent contains the components required for the first measurement principle, by providing a line 3 which connects the pump 18 and the first flow path switching unit 141, the pump 142 can be used to aspirate the first reagent. In those cases where the components required for the first measurement principle are added to the third reagent, by providing a line 4 that connects the pump 23 and the first flow path switching unit 141, the pump 142 can be used to aspirate the third reagent. The method used for mixing the first and third reagents with the blood sample may be the same as the method used for mixing the second reagent and the blood sample.

The second type of method refers to a method in which glucose is reacted with a specific enzyme in the presence of ATP (adenosine triphosphate), subsequent reaction of the obtained substance with another enzyme converts NADP (the oxidized form of nicotinamide adenine dinucleotide phosphate) to NADPH (the reduced form of nicotinamide adenine dinucleotide phosphate), and the increase in the amount of NADPH is measured by the change in absorbance. Examples of the enzymes typically used for reaction with glucose include glucose-6-phosphate dehydrogenase (G6-PDH). Examples of this type of method include the hexokinase-G6-PDH method and the glucokinase-G6-PDH method. In other words, in the case of this second measurement principle, at least ATP, two enzymes and NADP are required, and these components are included in the reagent added to the blood sample when measuring the second measurement item, namely the reagent containing the second reagent and at least one of an eluent (the first reagent or the third reagent) and a diluent.

NADPH has a peak absorption at 340 nm. When whole blood is used as the blood sample, because the NADPH absorption spectrum overlaps with the absorption spectrum of the various forms of hemoglobin including HbA1c, detection of NADPH can sometimes be difficult. Further, the produced NADPH can be detected by colorimetry or fluorescence using additional conventiona methods, but the reagent composition becomes complex. Accordingly, in terms of simplifying the reagent composition and ensuring superior reagent storage stability, the first measurement principle is preferred as the glucose measurement principle. Among the various methods that employ the first measurement principle, the GOD-POD method or the mutarotase-GOD-POD method is preferred.

In those cases where a color developer is colored by using hydrogen peroxide and POD, a phenol or an aniline is preferably used as the color developer. The phenol or aniline functions as a hydrogen donor. Phenol-based hydrogen donors are generally referred to as Trinder's reagents. Aniline-based hydrogen donors are generally referred to as modified Trinder's reagents (see Tamaoku et al., "New Water-soluble Hydrogen Donors for the Enzymatic Photometric Determination of Hydrogen Peroxide. II. N-Ethyl-N-(2-hydroxy-3-sulfopropyl)aniline Derivatives", Chem. Pharm. Bull. 30(7) 2492 to 2497, 1982). The Trinder's reagent used in the present invention may be a modified Trinder's reagent. In the color development step, a modified Trinder's reagent may be used instead of a Trinder's reagent. The Trinder's reagent undergoes a condensation reaction with a coupling agent such as 4-aminoantipyrine (also abbreviated a 4-AA) or 3-methyl-2-benzothiazoline hydrazone and is converted to a colorant having a specific peak absorption wavelength.

Examples of the color developer include phenol, phenol derivatives, Trinder's reagents and modified Trinder's reagents. Examples of the modified Trinder's reagents include ADOS (CAS number [82692-96-4], an oxidative condensation colorant with 4-AA having an absorption peak wavelength of 542 nm), ADPS (CAS number [82611-88-9], an oxidative condensation colorant with 4-AA having an absorption peak wavelength of 540 nm), ALPS (CAS number [82611-85-6], an oxidative condensation colorant with 4-AA having an absorption peak wavelength of 561 nm), DAOS (CAS number [83777-30-4], an oxidative condensation colorant with 4-AA having an absorption peak wavelength of 593 nm), HDAOS (CAS number [82692-88-4], an oxidative condensation colorant with 4-AA having an absorption peak wavelength of 583 nm), MAOS (CAS number [82692-97-5], an oxidative condensation colorant with 4-AA having an absorption peak wavelength of 630 nm), TOOS (CAS number [82692-93-1], an oxidative condensation colorant with 4-AA having an absorption peak wavelength of 555 nm), TOPS (CAS number [40567-80-4], an oxidative condensation colorant with 4-AA having an absorption peak wavelength of 550 nm), MADB (CAS number [209518-16-1], an oxidative condensation colorant with 4-AA having an absorption peak wavelength of 630 nm), and TODB (CAS number [1044537-70-3], an oxidative condensation colorant with 4-AA having an absorption peak wavelength of 550 nm).

In terms of reducing the effects from the absorption spectra of the plasma component and hemoglobin contained in the blood sample, the modified Trinder's reagent is preferably a reagent which yields a compound upon reaction with the coupling agent having an absorption peak wavelength of 550 nm or higher, and more preferably within a range from 580 to 800 nm. Examples of reagents that yield a color development peak within this range from 580 to 800 nm include DAOS, HDAOS, MAOS and MADB.

The second reagent may also contain components for buffering or controlling the pH, the ionic strength, or the osmotic pressure or the like. Examples of these components include acetic acid, citric acid, phosphoric acid, Tris, glycine, boric acid, carbonic acid, Good's buffers, as well as the sodium salts, potassium salts, and calcium salts and the like of these components.

The pH of the second reagent may be selected as appropriate based on the optimal pH for the enzyme, and the best pH for ensuring suitable color development of the colorant.

In some cases, the second reagent preferably also contains a salt. The salt is preferably an inorganic salt. Examples of the inorganic salt include sodium chloride and potassium chloride. The concentration of the buffer and the salt may be any concentration that allows satisfactory reaction of the enzyme, and may be selected as appropriate in accordance with the type of enzyme being used.

The second reagent may also contain a component that stabilizes the enzyme or the colorant. Examples of components that stabilize the enzyme or the colorant include proteins such as casein and bovine serum albumin, polymer compounds such as polyethylene glycol, polyvinylpyrrolidone and phospholipid polymers, as well as sugars, surfactants, oxidizing agents, reducing agents and chaotropic agents. A single component that stabilizes the enzyme or the colorant may be used, or a combination of a plurality of such components may be used.

The second reagent may also contain a specific compound, enzyme, or surfactant or the like for removing components that affect the measurements. Examples of these types of compounds include ferrocyanide ions (Japanese Unexamined Patent Application, First Publication No. Sho 55-138656) and EDTA-iron complexes (Japanese Unexamined Patent Application, First Publication No. Sho 57-71398). Examples of these types of enzymes include ascorbate oxidase, bilirubin oxidase and catalase. Examples of these types of surfactants include amphoteric surfactants (Japanese Unexamined Patent Application, First Publication No. Hei 07-155196) and nonionic surfactants (International Patent Publication No. 2013/147309).

When the measurement target substance is glucose, the compounds described above required for the measurement may undergo mutual reaction in a solution state. In order to prevent fluctuations in the amount of each component over long periods, the above components may be divided among a plurality of solutions. For example, in those cases where the second reagent contains a Trinder's reagent, in order to ensure that a condensation reaction does not occur between the coupling agent and the Trinder's reagent during storage in the second reagent holder 13, the coupling agent and the Trinder's reagent are preferably prepared as mutually separate reagents.

For example, the coupling agent may be included in either one of the eluent (the first reagent or the third reagent) and the diluent, with the Trinder's reagent included in the second reagent. Alternatively, the Trinder's reagent may be included in either one of the eluent (the first reagent or the third reagent) and the diluent, with the coupling agent included in the second reagent. In one specific example, in the case of a reagent prepared by the mutarotase-GOD-POD method, the 4-AA that functions as the coupling agent may be included in the diluent, and the Trinder's reagent, the mutarotase and the peroxidase may be included in the second reagent. When the blood sample is mixed with the diluent, the blood sample and the 4-AA are mixed. By subsequently adding the second reagent, the Trinder's reagent, the mutarotase and the peroxidase are added, thus enabling the enzyme reaction to proceed and the glucose concentration-dependent color development to occur.

The amount of glucose concentration-dependent color development is measured by the flow cell optical detector 17. The measurement method may employ any appropriate method, and for example, following mixing of the blood sample and the second reagent and subsequent elapsing of a certain time, the increase in absorbance per unit of time may be measured using the rate method. Further, the difference between the absorbance immediately following mixing of the blood sample and the second reagent and the absorbance following a certain period of time may be measured using the end point method. When measurement is conducted by the end point method, an additional step may be conducted in which, prior to introducing the mixed liquid of the blood sample and the second reagent into the flow cell optical detector 17, solutions of the required components among the eluent, the diluent and the second reagent are passed through the flow cell optical detector 17, with a blank absorbance being measured for each of these solutions.

Examples of the combination of at least one of the eluent and the diluent with the components contained in the second reagent are shown in (1) to (8) of Table 2. In Table 2, the "at least one of the eluent and the diluent" is recorded as simply the "eluent or diluent". Further, in Table 2, the "enzyme" refers to at least one enzyme selected from glucose oxidase (GOD), mutarotase and pyranose oxidase. Those skilled in the art will be able to select appropriate combinations with due consideration of factors such as the stability of the enzyme used and the mutual interactions between components. Among the various options, in order to better prevent contamination and degradation of the liquid chromatography column, the components added to at least one of the eluent and the diluent are preferably added to the diluent, and combination 4 or 8 is particularly preferred. Reagents containing an enzyme and a Trinder's reagent are preferably kept at a stable temperature by the second temperature adjustment mechanism.

**[Table 2]**

| Combination | 1 | 2 | 3 | 4 | 5 | 6 | 7 | 8 |
|---|---|---|---|---|---|---|---|---|
| Eluent or diluent | Enzyme POD Trinder's reagent | Enzyme Trinder's reagent | POD Trinder's reagent | Trinder's reagent | Enzyme POD Coupling agent | Enzyme Coupling agent | POD Coupling agent | Coupling agent |
| Second reagent | Coupling agent | POD Coupling agent | Enzyme Coupling agent | Enzyme POD Coupling agent | Trinder's reagent | POD Trinder's reagent | Enzyme Trinder's reagent | Enzyme POD Trinder's reagent |

The components required for the glucose measurement may be divided and added to two reagents; namely either one of the eluent (the first reagent or third reagent) and the diluent, and the second reagent. In those cases where the components required for the glucose measurement are divided and added to two reagents, the two reagents are preferably the diluent and the second reagent. Further, the components required for the glucose measurement may also be divided and added to three reagents. In those cases where the components required for the glucose measurement are divided and added to three reagents, the three reagents may be composed of two reagents selected from among the first reagent, the third reagent and the diluent, together with the second reagent. In those cases where the components required for the glucose measurement are divided and added to three reagents, the three reagents are preferably the third reagent, the diluent and the second reagent.

The above description describes examples in which, when the components required for measurement of the second measurement item are to be added to a separate reagent from the second reagent, those components required for measurement of the second measurement item are added to reagents used for other purposes, such as the diluent, the first reagent and the third reagent.

In another aspect, in order to prevent mutual reaction in the solution state of the above components required for the various measurements, the components required for measurement of the second measurement item may be divided and used to prepare the second reagent and a fourth reagent. For example, the components required for measurement of the glucose may be divided between the three solutions of the second reagent, the fourth reagent and the diluent.

The fourth reagent contains mainly a reagent for measuring the second measurement item, namely for measuring glucose in the example described above. The types of components that may be included in the fourth reagent are the same as those of the second reagent, and therefore description of those components is omitted here.

In one example, in the case of the GOD-POD method, the coupling agent 4-AA, peroxidase, glucose oxidase and a Trinder's reagent are used as the reagents. Each of these components may be added to one of the three solutions among the second reagent, the fourth reagent and the diluent with due consideration of the stability of these four reagents and the various interactions between the components. For example, the reagents can be prepared by adding the coupling agent 4-AA to the diluent, the peroxidase to the second reagent, and the glucose oxidase and the Trinder's reagent to the fourth reagent. When the blood sample is mixed with the diluent, the blood sample and the 4-AA are mixed. By then adding the second reagent to this mixture, the peroxidase is added, and by then adding the fourth reagent, the glucose oxidase and the Trinder's regent are added, enabling the enzyme reaction to proceed and the glucose concentration-dependent color development to occur.

In the case of the GOD-POD method, the combination of components contained in the second reagent, the fourth reagent and the diluent may be selected appropriately with due consideration of the stability of the enzyme being used, and the various interactions between the components including the ferrocyanide ions, EDTA-iron complexes, ascorbate oxidase and bilirubin oxidase mentioned above. Among the various options, in order to prevent contamination and degradation of the liquid chromatography column, the enzyme and the Trinder's reagent are preferably included in the second reagent or the fourth reagent. Further, ferrocyanide ions are known to interact with various other components. In those cases where ferrocyanide ions are added, the Trinder's reagent in particular is preferably added to a different reagent from the ferrocyanide ions. Specific examples of the combination of components included in the second reagent, the fourth reagent and the diluent include (1) to (6) in Table 3 below. Of these, combination (4) is preferred. Reagents containing an enzyme and a Trinder's reagent are preferably kept at a stable temperature by the second temperature adjustment mechanism.

**[Table 3]**

| Combination | 1 | 2 | 3 | 4 | 5 | 6 |
|---|---|---|---|---|---|---|
| Second reagent | GOD Ferrocyanide ions | GOD Trinder's reagent | GOD POD Ferrocyanide ions | POD Ferrocyanide ions | POD Trinder's reagent | Trinder's reagent |
| Fourth reagent | POD Trinder's reagent | POD Ferrocyanide ions | Trinder's reagent | GOD Trinder's reagent | GOD Ferrocyanide ions | GOD POD Ferrocyanide ions |
| Diluent | Coupling agent | Coupling agent | Coupling agent | Coupling agent | Coupling agent | Coupling agent |

In those cases where HbA1c is separated using the liquid chromatography column 16, whole blood is generally dilutes approximately 101-fold. In the measurement of the glucose concentration, the blood sample is generally diluted to a final value of approximately 80-fold for measurement. Because the dilution factors for the blood sample used in the HbA1% measurement and the glucose concentration measurement are similar, the two measurements are quite compatible.

In those cases where the second measurement item is the glycoalbumin %, both the total albumin and glycoalbumin are measured, and the percentage of glycoalbumin relative to the total albumin is then calculated. In those cases where the glycoalbumin % is determined by high-performance liquid chromatography, the albumin and glycoalbumin may be separated using the liquid chromatography column 16, or may be separated using a second liquid chromatography column connected to the second line 25.

In those cases where the total albumin is measured using a biochemical method, measurement may be conducted using conventional regents such as the BCG (bromocresol green) method or the modified BCP (bromocresol purple) method.

In those cases where the glycoalbumin is measured using an enzyme method, measurements may be conducted, for example, using the steps described below. (1) the glycoalbumin in the blood sample is digested using a protease to form fructosylamino acid. (2) Using this fructosylamino acid as a matrix, a reaction is conducted with a keto amino oxidase or fructosamine oxidase in the presence of oxygen to generate hydrogen peroxide. (3) This hydrogen peroxide is reacted with POD in the presence of a color developer to achieve color development, and the glycoalbumin is then quantified based on an absorbance measurement.

In those cases where the second measurement item is 1,5-AG, the reagent used for measuring the 1,5-AG may be an enzyme method reagent, and the measurement may be conducted, for example, using the steps described below. (1) ADP (adenosine diphosphate)-dependent hexokinase and adenosine-5'-diphopshate are brought into contact with the 1,5-AG in the specimen, producing 1,5-AG6-phosphate. (2) The 1,5-AG6-phosphate and NADP are reacted with 1,5-AG dehydrogenase to produce NADPH. (3) This NADPH is detected and used to quantify the 1,5-AG.

When preparing the mixed liquid, in order to accelerate color development reaction, the temperature is preferably maintained at a temperature within a range from 20 to 50°C using the first temperature adjustment mechanism 28 illustrated in FIG. 8.

The HbA1c% separated in the liquid chromatography column 16 is measured using the flow cell optical detector 17. Specifically, HbA1% is calculated from the optical density (also known as the OD value) in the wavelength band from 300 to 600 nm in which hemoglobins exhibit absorption. Hemoglobins exhibit characteristic absorptions within a range from 350 to 450 nm, and within a range from 520 to 590 nm. Accordingly, the measurement wavelength is preferably within a range from 350 nm to 450 nm or from 520 to 590 nm, is more preferably from 350 to 450 nm, and is most preferably within a range from 380 to 425 nm.

Following measurement of the HbA1c concentration, the first flow path switching unit 141 is switched so as to connect the introduction section 11 and the pump 142. The mixed liquid of the blood sample and the second reagent is then aspirated from the introduction section 11 using the pump 142. Subsequently, the mixed liquid passes through the injection port 143 and is introduced into the first line 15, and the glucose concentration of the mixed liquid is measured by the flow cell optical detector 17. In the case of Variation 1, following aspiration of the mixed liquid of the blood sample and the second reagent using the pump 142, the first flow path switching unit 141 links the pump 142 and the second line 25, the mixed liquid is introduced into the second line 25, and the glucose concentration of the mixed liquid is measured by the flow cell optical detector 17. The measurement wavelength used in the flow cell optical detector 17 may be set in accordance with the color development peak within the mixed liquid based on the second reagent being used.

The OD value corresponding with the glucose concentration measured using the method described above may sometimes yield a higher value than the actual glucose concentration due to the effect of absorbance by hemoglobin or the like contained in the mixed liquid. Further, the glucose concentration is calculated as the glucose mass relative to the volume of blood plasma, but because there are individual differences in the mass ratio between the blood cell component and the plasma component in whole blood, the amount of the plasma component contained in the sample set in the introduction section 11 may sometimes differ from sample to sample. In order to avoid these effects, a correction is preferably applied for the OD value corresponding with the concentration of the hemoglobin or the like.

One example of the correction method involves applying a correction based on the total hemoglobin area of the chromatography of each hemoglobin separated by the liquid chromatography column 16. The hemoglobin absorption coefficient at the measurement wavelength for each hemoglobin is well known. By using this absorption coefficient and the total hemoglobin area, the hemoglobin-derived absorbance value at the wavelength used for measuring the glucose concentration can be calculated, and by subtracting the hemoglobin-derived absorbance value from the absorbance value observed when measuring the glucose, the OD value can be corrected.

Furthermore, the degree of divergence between the actual glucose concentration and the OD value sometimes differs between the case when the glucose concentration is low and the case when the glucose concentration is high. In such cases, a correction may be applied by calculating a regression formula based on the wavelength at which the OD value fluctuates depending on the total hemoglobin contained in the blood sample, and the OD value at the wavelength at which the glucose concentration is measured. Using this regression formula as a correction formula, the OD value corresponding with the glucose concentration of the blood sample can be corrected. The regression formula may be a linear formula, a quadratic formula or a cubic formula. Further, the regression formula may employ a plurality of formulas for each hemoglobin amount.

As described above, the blood analysis method according to one aspect of the present invention enables the measurement of a first component and a second component in a blood sample using a single blood sample and a single flow cell optical detector. Accordingly, the method enables two different components to be measured in a simple manner.

### Examples

The present invention is described below in further detail using a series of examples, but the present invention is not limited by the following description.

### [Example 1]

Using an HPLC device having an absorbance meter as a flow cell optical detector, the glucose concentration of a series of samples was measured.

### 1. Sample Preparation

Glucose aqueous solutions A having glucose concentrations of 0, 300, 600, 1,200, 1,800, 2,400, and 3,000 mg/dL were prepared. Whole blood samples were collected from volunteers, and following refrigerated storage for at least one week from collection, the whole blood samples and the above glucose aqueous solutions A were mixed at a ratio of 5:1, completing preparation of whole blood samples having glucose concentrations of 0, 50, 100, 200, 300, 400 and 500 mg/dL (also referred to as glucose-added whole blood samples). In separate preparations, the above glucose aqueous solutions A and purified water were mixed at a ratio of 5:1 to prepare glucose aqueous solutions B having glucose concentrations of 0, 50, 100, 200, 300, 400 and 500 mg/dL.

### 2. Preparation of Glucose Measurement Reagent

Glucose CII Test Wako (manufactured by FUJIFILM Wako Pure Chemical Corporation) which uses the mutarotase-GOD-POD method was used as the glucose measurement reagent. Using 17.5 mL of a buffer solution (1) containing a phosphate buffer (pH 7.1) and phenol as a color developer, a color developer (2) containing mutarotase, GOD, POD, 4-aminoantipyrine and ascorbate oxidase was dissolved (glucose measurement reagent A). The concentration was 20 times that noted in the documentation provided with the reagent.

### 3. Glucose Measurement Reaction

Three µL samples of each of the glucose-added whole blood samples and glucose aqueous solutions B were diluted 101-fold by adding 300 µL of a solution prepared by adding phenol to a hemolysis washing solution for RC20 manufactured by Sekisui Medical Co., Ltd. in an amount sufficient to produce a final concentration of 0.1% by weight, thus preparing measurement samples. Next, 15 µL of the glucose measurement reagent A was added to each measurement sample, and the resulting mixture was left to stand at 37°C for 5 minutes, thus obtaining a reaction liquid. The hemolysis washing solution for RC20 is a solution capable of hemolysis of the red blood cells in whole blood.

### 4. HPLC Measurement

Using an HPLC device (product number: LC20A, manufactured by Shimadzu Corporation), measurement of the mixed liquids was conducted without fitting a liquid chromatography column to the device. Using an eluent A for use with the RC20 glycohemoglobin analyzer manufactured by Sekisui Medical Co., Ltd. as the eluent, measurements were conducted under conditions including an injection volume of the above reaction liquid of 5 µL, a flow rate of 1.1 mL/min., and a measurement wavelength of 505 nm.

### 5. Results and Observations

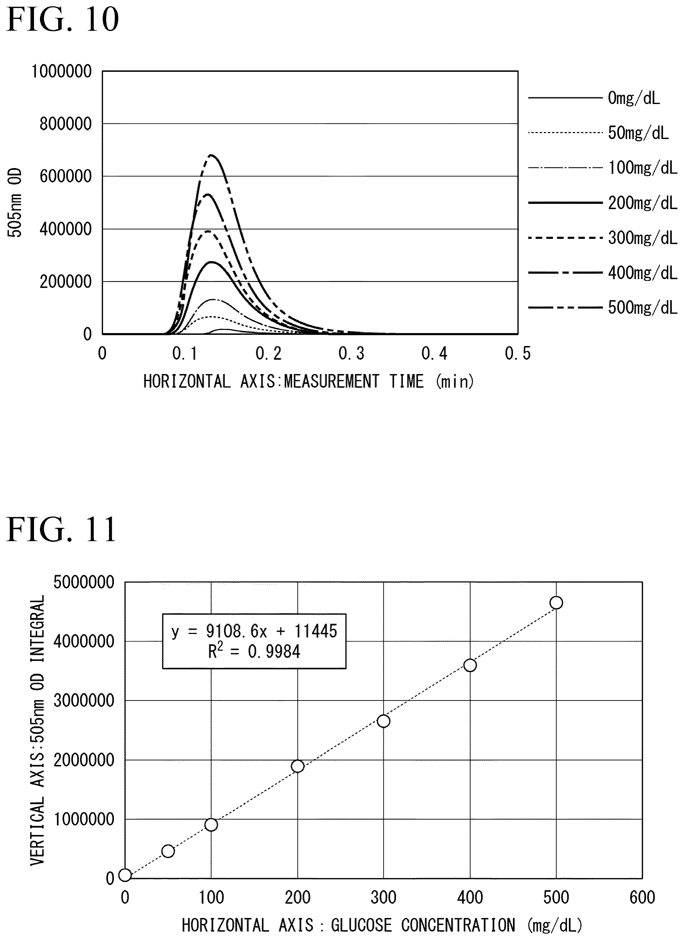

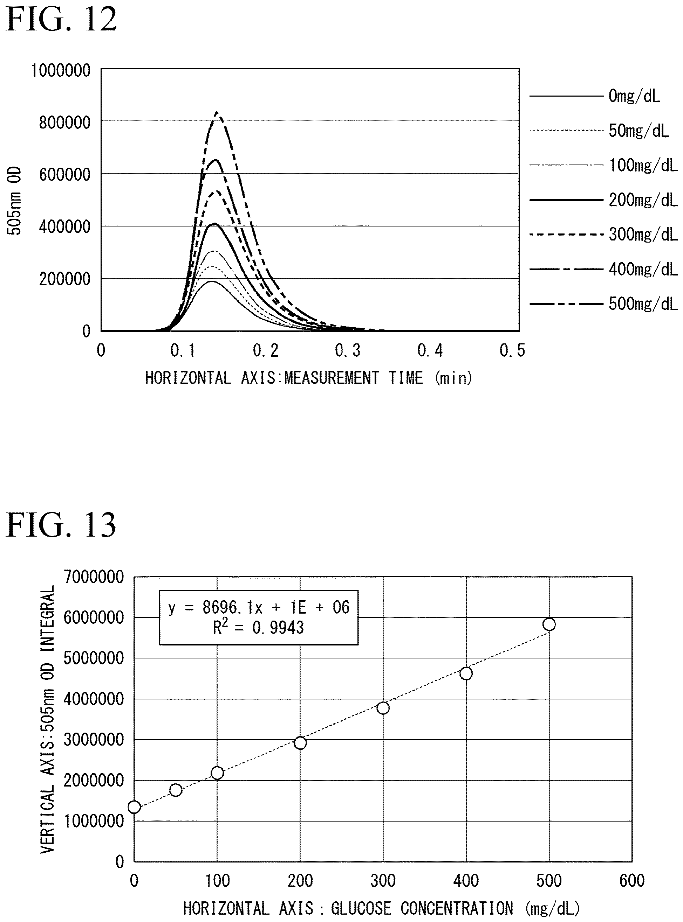

FIG. 10 is a graph illustrating the OD value at 505 nm plotted against the measurement time during measurements conducted using the glucose aqueous solutions B as samples. FIG. 11 is a graph illustrating the integral of the OD value at 505 nm plotted against the glucose concentration during measurements conducted using the glucose aqueous solutions B as samples. FIG. 12 is a graph illustrating the OD value at 505 nm plotted against the measurement time during measurements conducted using the glucose-added whole blood samples as samples. FIG. 13 is a graph illustrating the integral of the OD value at 505 nm plotted against the glucose concentration during measurements conducted using the glucose-added whole blood samples as samples.

The graph of FIG. 11 exhibits good linearity, confirming that the glucose concentration could be measured by using the flow cell optical detector of the HPLC device to measure mixed liquids of the glucose aqueous solutions B and the glucose measurement reagent A.

In the graph of FIG. 13, an increase in the blank due to the hemoglobin in the whole blood samples was observed, but the linearity was good. Accordingly, it was evident that the glucose concentration in the whole blood samples could be measured using the flow cell optical detector of the HPLC device.

### [Example 2]

In order to verify a glucose measurement method that was substantially unaffected by the components other than glucose contained in whole blood, the color developer in the glucose measurement reagent was changed to MAOS.

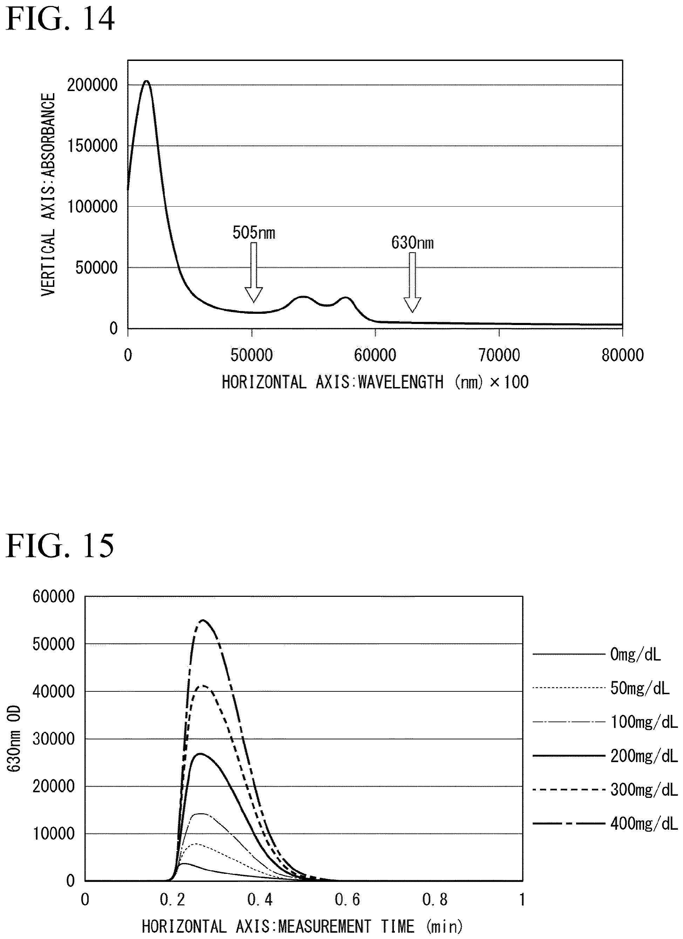

FIG. 14 illustrates an absorption spectrum of whole blood diluted 101-fold with the hemolysis washing solution for RC20 manufactured by Sekisui Medical Co., Ltd. (measured using an LC20A manufactured by Shimadzu Corporation as the HPLC device). The whole blood absorption spectrum can be said to have low absorbance at wavelengths of 600 nm or higher. Accordingly, MAOS which yields a color development peak at a wavelength higher than 600 nm in a mixed liquid with glucose was used as the color development matrix.

### 1. Sample Preparation

Whole blood samples prepared in the same manner as Example 1 were used, with glucose concentrations of 0, 50, 100, 200, 300, 400, 500 and 1,000 mg/dL (also referred to as glucose-added whole blood samples).

### 2. Preparation of Glucose Measurement Reagent

A glucose measurement reagent B was prepared with the composition shown below. MAOS was procured from Dojindo Laboratories Co., Ltd. Further, the various enzymes used commercially procured products.
- 20 mM phosphate buffer (pH 7.2)
- 4-aminoantipyrine : 85 mg/mL
- MAOS: 40 mg/mL
- glucose oxidase: 340 units/mL
- peroxidase: 350 units/mL
- ascorbate oxidase: 60 units/mL
- mutarotase: 100 units/mL

### 3. Glucose Measurement Reaction

Three µL samples of each of the glucose-added whole blood samples were diluted 101-fold by adding the hemolysis washing solution for RC20 manufactured by Sekisui Medical Co., Ltd., thus preparing measurement samples. Next, 15 µL of the glucose measurement reagent B was added to each measurement sample, and the resulting mixture was left to stand at 37°C for 5 minutes, thus obtaining a reaction liquid.

### 4-1. HPLC Measurement-1

With the exception of altering the measurement wavelength to 630 nm, measurements were conducted in the same manner as Example 1. At this wavelength, a substance produced by the binding of MAOS to the 4-aminoantipyrine coupling agent can be measured.

### 4-2. HPLC Measurement-2

Using an HPLC device (product number: RC20, manufactured by Sekisui Medical Co., Ltd.), measurement of the mixed liquids was conducted without fitting a liquid chromatography column to the device. The eluent A for use with the RC20 was used as the eluent. Measurements of the glucose-added whole blood samples were conducted under conditions including an injection volume of 4 µL, a flow rate of 0.6 mL/min., and a measurement wavelength of 660 nm. At this wavelength, as above, a substance produced by the binding of MAOS to the 4-aminoantipyrine coupling agent can be measured.

### 5. Results and Observations

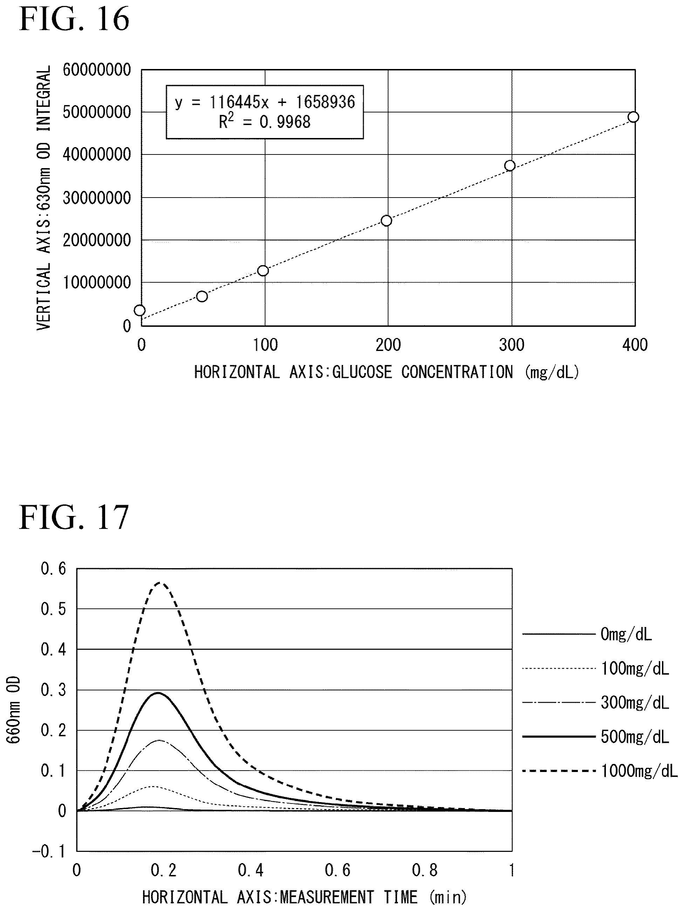

FIG. 15 is a graph illustrating the OD value at 630 nm plotted against the measurement time during measurements conducted using the glucose-added whole blood samples as samples. FIG. 16 is a graph illustrating the integral of the OD value at 630 nm plotted against the glucose concentration of the whole blood samples.

In FIG. 13, a blank (intercept) of a value corresponding with a glucose concentration of approximately 148 mg/dL occurred, but in FIG. 16, the value for the blank, when converted to an equivalent glucose concentration, has decreased to about 14 mg/dL. It is thought that this intercept is due to components such as hemoglobin in the whole blood samples. These results reveal that the use of a color development matrix such as MAOS, having a color development peak for a mixed liquid with glucose at 600 nm or higher, is effective in suppressing any increase in the blank. FIG. 16 exhibits good linearity, and therefore it was evident that even in those cases where MAOS is used as the color developer, the glucose concentration in whole blood samples could be measured using the flow cell optical detector of the HPLC device.

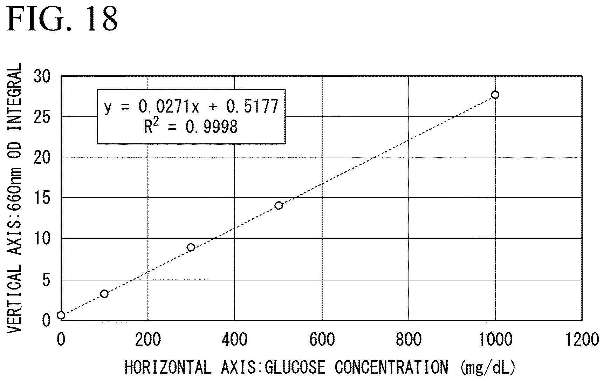

FIG. 17 is a graph illustrating the OD value at 660 nm plotted against the measurement time during for the glucose-added whole blood samples. FIG. 18 is a graph illustrating the integral of the OD value at 660 nm plotted against the glucose concentration of the glucose-added whole blood samples.

In the graph of FIG. 18, the increase in the blank, which is thought to be due to the whole blood, when converted to an equivalent glucose concentration was approximately 19 mg/dL. Further, the graph exhibits good linearity, confirming that the glucose concentration contained in the whole blood samples could be measured using the absorbance meter that represents the detector of the HPLC device. Based on the above results, it was evident that by using the absorbance meter that functioned as the detector for the HPLC device, the glucose concentration of whole blood samples could be measured without being restricted to a specific HPLC device.

### [Example 3]

A reagent combination was tested in which 4-aminoantipyrine was included in the diluent, and a reagent containing mutarotase, GOD, POD and a Trinder's reagent was used as the second reagent. Whole blood samples were diluted with the diluent containing 4-aminoantipyrine, HPLC measurements were then conducted, and the effects of the 4-aminoantipyrine on the measurement of HbA1c% were evaluated.

### 1. Preparation of Reagents and Samples

First, 4-aminoantipyrine was added to the hemolysis washing solution for RC20 in sufficient amount to achieve a final concentration of 1 mmol/L (hereinafter referred to as the 4AA-added diluent). Next, 300 µL of this 4AA-added diluent was added to a series of 3 µL whole blood specimens collected from three volunteers, thus producing blood samples. Blood samples obtained by diluting the whole blood specimens to the same dilution ratio using the hemolysis washing solution for RC20 were also prepared as controls. The whole blood of the three different donors was investigated. No 4-aminoantipyrine was added to the hemolysis washing solution for RC20.

### 2. HPLC Measurement

An HPLC device (product number: RC20, manufactured by Sekisui Medical Co., Ltd.) was fitted with an RC20 column, and the diluted whole blood specimens (namely, the blood samples) were measured using the eluent A for RC20 as the first reagent and the eluent B for RC20 as the third reagent. The measurements were conducted under conditions including an injection volume for the diluted specimens of 4 µL, a flow rate of 1.1 mL/min., and a gradient conforming with the RC20 program.

### 3. Results and Observations

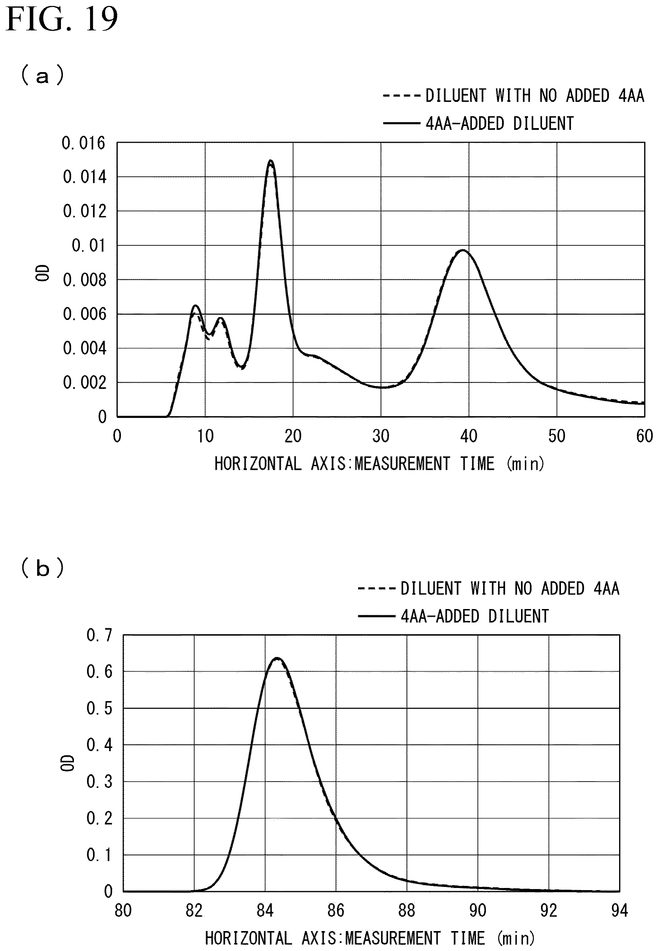

FIG. 19 is a chromatogram plotting the results measured using the RC20. The vertical axis represents the absorbance (OD value) obtained by subtracting the absorbance at 660 nm from the absorbance at 415 nm. The HbA1c peak occurs at a retention time of about 39 seconds as illustrated in FIG. 19(a), whereas the HbA0 peak occurs a retention time of about 84 seconds as illustrated in FIG. 19(b). The chromatograms for each of the hemoglobins including HbA1c were similar for the case using the hemolysis washing solution for RC20 with no added 4AA, and the case using the 4AA-added diluent.

Table 4 shows the results of calculating the HbA1c% measurement values from the chromatograms. The HbA1% measurement value in the case where the typical hemolysis washing solution for RC20 was used, and the HbA1c% measurement value in the case where the 4AA-added diluent was used were very consistent for the specimens from all three volunteers.

**[Table 4]**

| | HbA1% measurement results (%) | | | | | |
|---|---|---|---|---|---|---|
| | Hemolysis washing solution for RC20 | | | 4-aminoantipyrine-added diluent | | |
| | Specimen 1 | Specimen 2 | Specimen 3 | Specimen 1 | Specimen 2 | Specimen 3 |
| First measurement | 5.73 | 5.56 | 4.42 | 5.75 | 5.56 | 4.41 |
| Second measurement | 5.74 | 5.57 | 4.42 | 5.74 | 5.57 | 4.40 |
| Average value | 5.74 | 5.57 | 4.42 | 5.75 | 5.57 | 4.41 |

Based on FIG. 19 and Table 4, it was evident that the addition of 4-aminoantipyrine to the diluent has no effect on the HbA1c% measurement, and that even when the diluent contains a component for measuring glucose, the HbA1c% can still be measured with excellent accuracy.

The above results indicate that when a blood sample is diluted with a diluent containing a component for measuring glucose and then introduced into a liquid chromatography column, the hemoglobins can be separated and a flow cell optical detector can be used to measure HbA1c% with good accuracy, and that when the component for measuring glucose is added to the dilute specimen, the same flow cell optical detector can be used to accurately measure the glucose concentration.

### Industrial Applicability

By employing the aspects described above, a blood analysis device and a blood analysis method with a simplified detection mechanism can be provided.

### Reference Signs List

1, 1', 2, 2', 3, 3', 4 and 5: Blood analysis device; 11: Introduction section; 12: First reagent holder; 13: Second reagent holder; 14: Liquid delivery control mechanism; 15: First line; 16: Liquid chromatography column; 17: Flow cell optical detector; 18, 23, 24, 27, 142: Pump; 19: Waste liquid tank; 20: Control unit; 21: Third reagent holder; 22: Mixer; 25: Second line; 26: Diluent holder; 28: First temperature adjustment mechanism; 29: Second temperature adjustment mechanism; 30: Fourth reagent holder; 141: First flow path switching unit; 143: Injection port; 144: Second flow path switching unit; 145: Third flow path switching unit; and 146: Fourth flow path switching unit

## Claims

1. A blood analysis device comprising:
an introduction section into which a blood sample is introduced,
a first reagent holder in which a first reagent for measuring a first measurement item of the blood sample is stored,
a first pump that controls liquid delivery of the first reagent,
a second reagent holder in which a second reagent for measuring a second measurement item of the blood sample is stored,
a liquid delivery control mechanism which is connected to the introduction section, the first pump and the second reagent holder, and controls liquid delivery of the blood sample and the second reagent,
a first line which is connected to the liquid delivery control mechanism, and into which at least a portion of the blood sample is introduced,
a liquid chromatography column which is connected to the first line, and
a flow cell optical detector which is connected to the liquid chromatography column and measures the first measurement item and the second measurement item.

2. The blood analysis device according to Claim 1, further comprising:
a second line which connects the liquid delivery control mechanism and the flow cell optical detector.

3. The blood analysis device according to Claim 2, wherein
the liquid delivery control mechanism comprises a flow path switching unit between the introduction section and the second line,
the second line connects the flow path switching unit and the flow cell optical detector, and
the flow path switching unit can be switched to block connection between the introduction section and the liquid chromatography column, and connect the introduction section and the second line.

4. The blood analysis device according to Claim 3, wherein
the liquid delivery control mechanism further comprises a second pump connected to the flow path switching unit, and an injection port positioned between the flow path switching unit and the introduction section.

5. The blood analysis device according to Claim 1 or 2, wherein the flow cell optical detector detects absorbance.

6. The blood analysis device according to Claim 1 or 2, further comprising:
a third reagent holder in which a third reagent for measuring the first measurement item is stored,
a third pump which controls liquid delivery of a third reagent, and
a mixer which is connected to the first pump and the third pump, and mixes the first reagent and the third reagent, wherein
the mixer is connected to the liquid delivery control mechanism, and
a mixed liquid of the first reagent and the third reagent is supplied to the liquid chromatography column via the liquid delivery control mechanism.

7. The blood analysis device according to Claim 1 or 2, further comprising a diluent holder in which a reagent for diluting the blood sample is stored, wherein
the diluent holder is connected to the introduction section via the liquid delivery control mechanism.

8. The blood analysis device according to Claim 1 or 2, further comprising a first temperature adjustment mechanism for adjusting the temperature of at least the flow cell optical detector.

9. The blood analysis device according to Claim 8, further comprising a second temperature adjustment mechanism for adjusting the temperature of at least one of the first reagent holder and the second reagent holder.

10. The blood analysis device according to Claim 1 or 2, further comprising:
a fourth reagent holder in which a fourth reagent for measuring the second measurement item is stored, wherein
the fourth holder is connected to the liquid delivery control mechanism, and
the fourth reagent is introduced into the introduction section from the fourth reagent holder via the liquid delivery control mechanism.

11. The blood analysis device according to Claim 1 or 2, wherein the first measurement item is HbA1c%.

12. The blood analysis device according to Claim 1 or 2, wherein the second measurement item is glucose concentration.

13. A blood analysis method comprising:
a step of introducing a portion of a blood sample and a first reagent into a liquid chromatography column,
a step of separating a first component of the blood sample in the liquid chromatography column,
a step of measuring a concentration of the first component using a flow cell optical detector, and
a step of measuring a concentration of a second component in the blood sample using the flow cell optical detector, using a remainder of the blood sample and at least a second reagent.

14. The blood analysis method according to Claim 13, wherein the step of measuring the concentration of the second component using the flow cell optical detector comprises a step of preparing a mixed liquid by adding the second reagent to a remainder of the blood sample.

15. The blood analysis method according to Claim 14, wherein the step of preparing the mixed liquid is conducted either during or after the step of separating the first component.

16. The blood analysis method according to Claim 13 or 14, wherein the step of measuring the concentration of the second component using the flow cell optical detector is conducted after the step of measuring the first component.

17. The blood analysis method according to Claim 13 or 14, wherein the first component is HbA1c.

18. The blood analysis method according to Claim 13 or 14, wherein the second component is glucose.

19. The blood analysis method according to Claim 18, further comprising:
a step of calculating total hemoglobin from a chromatogram of the blood sample separated in the liquid chromatography column using the flow cell optical detector, and
a step of correcting the glucose concentration based on the total hemoglobin.

20. The blood analysis method according to Claim 13 or 14, wherein the blood sample is whole blood.

21. The blood analysis method according to Claim 13 or 14, wherein the step of measuring the concentration of the second component uses measurement principles of an enzyme method.

22. The blood analysis method according to Claim 13 or 14, wherein the step of measuring the concentration of the second component comprises a step of measuring an amount of hydrogen peroxide generated by a reaction of the second component and an enzyme.

23. The blood analysis method according to Claim 22, wherein a Trinder's reagent is used in the step of measuring the concentration of the second component.

24. The blood analysis method according to Claim 23, wherein the Trinder's reagent is a reagent having a color development peak within a range from 580 to 900 nm upon reaction with hydrogen peroxide.

25. The blood analysis method according to Claim 24, wherein the Trinder's reagent comprises MAOS (CAS number [82692-97-5]).

26. The blood analysis method according to Claim 23, wherein the Trinder's reagent is incorporated in the second reagent.

27. The blood analysis method according to Claim 13 or 14, wherein the step of introducing a portion of the blood sample and the first reagent into a liquid chromatography column comprises a step of diluting the blood sample with a diluent.

28. The blood analysis method according to Claim 21, wherein
the step of introducing a portion of the blood sample and the first reagent into a liquid chromatography column comprises a step of diluting the blood sample with a diluent, and
the diluent comprises at least one component selected from the group consisting of one or more enzymes used in the enzyme method reaction, color developers, and coupling agents.

29. The blood analysis method according to Claim 28, wherein the diluent comprises a coupling agent or a color developer.

30. The blood analysis method according to Claim 13 or 14 wherein in the step of measuring the concentration of the second component in the blood sample using the flow cell optical detector, a fourth reagent is also used.

31. The blood analysis method according to Claim 30, wherein
the step of measuring the concentration of the second component uses measurement principles of an enzyme method, and
the fourth reagent comprises at least one component selected from the group consisting of one or more enzymes used in the enzyme method reaction, color developers, and coupling agents.
